# EUROPÄISCHE PATENTSCHRIFT

(11) **EP 3 880 649 B1**
(45) Veröffentlichungstag und Bekanntmachung des Hinweises auf die Patenterteilung: **15.03.2023**
(21) Anmeldenummer: 19701816.1
(22) Anmeldetag: 23.01.2019
(51) Int. Cl.: C07C 69/675, C07C 67/03, C12P 7/62, A61P 3/02, A23L 33/12

(54) **VERFAHREN ZUR HERSTELLUNG VON POLYOLBASIERTEN ESTERN VON HYDROXYCARBONSÄUREN**
METHOD FOR PRODUCING POLYOL-BASED ESTERS OF HYDROXY CARBOXYLIC ACIDS
PROCÉDÉ DE PRÉPARATION D'ESTERS À BASE DE POLYOL D'ACIDES HYDROXYCARBOXYLIQUES

(30) Priorität: 17.01.2019 WO PCT/EP2019/051117
(43) Veröffentlichungstag der Anmeldung: 22.09.2021
(73) Patentinhaber: KetoLipix Therapeutics GmbH, 20459 Hamburg (DE)
(72) Erfinder: LOCHMANN, Dirk, 58453 Witten (DE); REYER, Sebastian, 58453 Witten (DE); STEHR, Michael, 58453 Witten (DE)
(74) Vertreter: Strehlke, Ingo Kurt
(86) Internationale Anmeldenummer: PCT/EP2019/051540
(87) Internationale Veröffentlichungsnummer: WO 2020/147979

(56) Entgegenhaltungen:
- WO-A1-2010/021766
- WO-A1-2013/150153
- WO-A2-2004/108740
- WO-A2-2006/012490
- US-A1- 2018 303 821
- G. BONORA ET AL: "Bakers Yeast Reduction of PEG-Linked Acetoacetate", LETTERS IN ORGANIC CHEMISTRY, Bd. 2, Nr. 1, 2005, Seiten 89-91, XP055627911, DOI: 10.2174/1570178053400135

## Beschreibung

Die vorliegende Erfindung betrifft das Gebiet der Ketokörper und des damit zusammenhängenden Stoffwechsels sowie die Therapie von damit im Zusammenhang stehenden Erkrankungen.

Insbesondere betrifft die vorliegende Erfindung ein Verfahren zur Herstellung von Polyolestern der 3-Hydroxybuttersäure, nämlich Polyglycerinestern der 3-Hydroxybuttersäure, sowie die auf diese Weise erhältlichen bzw. hergestellten Reaktionsprodukte (d. h. Polyolester der 3-Hydroxybuttersäure, nämlich Polyglycerinester der 3-Hydroxybuttersäure) und deren Verwendung, insbesondere in pharmazeutischen Zusammensetzungen, wie Arzneimitteln oder Medikamenten, oder in Nahrungsmittel- und/oder Lebensmittelerzeugnissen, sowie deren weitere Anwendungen bzw. Verwendungen.

Des Weiteren betrifft die vorliegende Erfindung pharmazeutische Zusammensetzungen, insbesondere Arzneimittel oder Medikamente, welche die nach dem erfindungsgemäßen Herstellungsverfahren erhältlichen bzw. hergestellten Reaktionsprodukte (d. h. Polyolester der 3-Hydroxybuttersäure, nämlich Polyglycerinester der 3-Hydroxybuttersäure) umfassen, sowie deren Anwendungen bzw. Verwendungen.

Schließlich betrifft die vorliegende Erfindung Nahrungsmittel- und/oder Lebensmittelerzeugnisse, insbesondere Nahrungsergänzungsmittel, funktionelle Lebensmittel *(Functional Food), Novel Food,* Lebensmittelzusatzstoffe, Nahrungszusätze, diätetische Lebensmittel, Power-Snacks, Appetitzügler und Kraft- und/oder Ausdauersport-Supplements, welche die nach dem erfindungsgemäßen Herstellungsverfahren erhältlichen bzw. hergestellten Reaktionsprodukte (d. h. Glyceride der 3-Hydroxybuttersäure) umfassen, sowie deren Anwendungen bzw. Verwendungen.

Im menschlichen Energiestoffwechsel ist Glucose der kurzfristig zur Verfügung stehende Energieträger, welcher in den Mitochondrien unter Freisetzung von Wasser und Kohlendioxid zu Energie verstoffwechselt wird. Bereits durch die Schlafperiode während der Nacht sind aber die Glycogenspeicher der Leber geleert. Jedoch benötigen vor allem das menschliche zentrale Nervensystem (ZNS) und das Herz eine permanente Energieversorgung.

Die physiologische Alternative zu Glucose, welche vor allem dem zentralen Nervensystem zur Verfügung steht, sind die sogenannten Ketokörper (synonym auch als Ketonkörper bezeichnet oder Englisch auch als *"Keton Bodies"* bezeichnet).

Der Begriff der Ketokörper ist insbesondere eine Sammelbezeichnung für drei Verbindungen, welche vor allem in katabolen Stoffwechsellagen (wie z. B. bei Hunger, Reduktionsdiät oder kohlenhydratarmer Ernährung) gebildet werden und unter Umständen zu einer Ketose führen. Unter den Begriff der Ketokörper fasst man insbesondere die drei Verbindungen Acetoacetat (synonym auch Acetacetat genannt) und Aceton sowie 3-Hydroxybuttersäure (nachfolgend synonym auch als beta-Hydroxybuttersäure oder BHB oder 3-BHB bezeichnet) bzw. deren Salz (d. h. 3-Hydroxybutyrat oder beta-Hydroxybutyrat) zusammen, wobei letztere Verbindung die bedeutendste der drei vorgenannten Verbindungen ist. 3-Hydroxybuttersäure bzw. deren Salz kommt physiologisch als (R)-Enantiomer vor, d. h. als (R)-3-Hydroxybuttersäure (synonym auch (3R)-3-Hydroxybuttersäure genannt, um das Chiralitätszentrum in 3-Position hervorzuheben) bzw. deren Salz.

Diese Ketokörper werden auch in großer Zahl beim Fasten oder Hungern physiologisch aus im Körper eingelagerten Lipiden durch Lipolyse bereitgestellt und ersetzen den Energieträger Glucose fast vollständig.

Die Ketokörper werden in der Leber aus Acetyl-Coenzym A (= Acetyl-CoA) gebildet, welches aus der beta-Oxidation stammt; sie stellen eine transportable Form des Acetyl-Coenzyms A im menschliche Körper dar. Zur Verwertung der Ketokörper müssen sich Gehirn und Muskeln aber zunächst umstellen, indem sie Enzyme exprimieren, welche zur Rückwandlung von Ketokörpern in Acetyl-Coenzym A benötigt werden. Insbesondere in Hungerzeiten tragen die Ketokörper einen beträchtlichen Anteil zur Energiegewinnung bei. So ist es beispielsweise dem Gehirn nach einiger Zeit möglich, mit nur einem Drittel der Tagesmenge an Glucose auszukommen.

Physiologisch erfolgt die Synthese der Ketokörper aus zwei Molekülen aktivierter Essigsäure in Form von Acetyl-Coenzym A, dem normalen Zwischenprodukt des Fettsäureabbaus, wobei zunächst mit Hilfe der Acetyl-Coenzym A-Acetyltransferase das Acetoacetyl-Coenzym A gebildet wird, welches unter Verwendung einer weiteren Acetyl-Coenzym A-Einheit und des Enzyms HMG-CoA-Synthase zum Zwischenprodukt 3-Hydroxy-3-methyl-glutaryl-CoA (HMG-CoA) verlängert wird, wobei schließlich die HMG-CoA-Lyase das Acetoacetat abspaltet. Diese drei Schritte finden ausschließlich in den Mitochondrien der Leber statt (Lynenzyklus), wobei 3-Hydroxybutyrat schließlich im Zytosol durch die D-beta-Hydroxybutyrat-Dehydrogenase entsteht. HMG-CoA ist außerdem ein Endprodukt beim Abbau der Aminosäure Leucin, während Acetoacetat beim Abbau der Aminosäuren Phenylalanin und Tyrosin entsteht.

Durch spontane Decarboxylierung entsteht aus Acetoacetat Aceton; es ist gelegentlich im Atem von Diabetikern und Diäthaltenden wahrzunehmen. Es kann vom Körper nicht weiterverwendet werden. Der Anteil von Aceton an den Ketokörpern ist allerdings gering.

Acetoacetat wird also reduktiv in die physiologisch relevante Form der 3-Hydroxybuttersäure bzw. des 3-Hydroxybutyrats überführt, kann aber auch unter Kohlenstoffdioxidfreisetzung in das physiologisch unbrauchbare Aceton zerfallen, was bei einer schweren Ketose, einer Ketoacidose (z. B. bei Diabetes mellitus Typ 1-Patienten ohne Insulinsubstitution), im Urin und in der Ausatemluft nachweisbar und olfaktorisch wahrnehmbar ist.

3-Hydroxybuttersäure wird derzeit im Bereich des Kraftsports als Natrium-, Magnesium- oder Calcium-Salz eingesetzt und in den Handel gebracht.

Jedoch ist 3-Hydroxybuttersäure für den Menschen evolutionär nicht oder in nur sehr geringer Menge bekannt, da Pflanzen keine 3-Hydroxybuttersäure produzieren und 3-Hydroxybuttersäure im tierischen Organismus nur bei toten ausgezehrten Tieren in der Ketose vorkommt, so dass 3-Hydroxybuttersäure bei peroraler Verabreichung Brechreiz auslöst. 3-Hydroxybuttersäure in Form der freien Säure sowie deren Salzen schmeckt zudem stark bitter und kann schweres Erbrechen und Übelkeit hervorrufen.

Zudem können Patienten, vor allem Neugeborene, aber auch Erwachsene größere Mengen an Salzen der 3-Hydroxybuttersäure nicht permanent verkraften, da diese Verbindungen nierenschädigend wirken können.

Außerdem ist die Plasmahalbwertszeit von 3-Hydroxybuttersäure und deren Salzen derart gering, dass selbst bei Einnahme von mehreren Gramm die Ketose nur für ca. drei bis vier Stunden vorhält, d. h. Patienten können insbesondere während der Nacht daher nicht von einer Therapie mit 3-Hydroxybuttersäure oder deren Salzen kontinuierlich profitieren. Bei Stoffwechselerkrankungen kann dies zu lebensbedrohlichen Situationen führen.

Daher werden im Fall der Therapie derartiger Stoffwechselerkrankungen heute sogenannte mittelkettige Triglyceride, sogenannte MCTs, für die ketogene Therapie eingesetzt, d. h. es wird die metabolische Umwandlung von Capron-, Capryl- und Caprinsäure (d. h. von gesättigten linearen C₆-, C₈- und C₁₀-Fettsäuren) aus den korrespondierenden Triglyceriden beabsichtigt.

Grundsätzlich stellt aber aus pharmazeutischer und klinischer Hinsicht 3-Hydroxybuttersäure demgegenüber ein wirksameres pharmazeutisch-pharmakologisches Zielmolekül, welches nach den Erkenntnissen des Standes der Technik prinzipiell für die Therapie einer Vielzahl von Erkrankungen zum Einsatz kommen könnte, aber aufgrund seiner mangelnden physiologischen Kompatibilität dort nicht zum Einsatz kommen kann (z. B. bei Erkrankungen im Zusammenhang mit einer Störung des Energiestoffwechsels, insbesondere Ketokörperstoffwechsels, oder neurodegenerativen Erkrankungen wie Demenz, Morbus Alzheimer, Morbus Parkinson etc., Fettstoffwechselerkrankungen usw.).

Die nachfolgende Tabelle veranschaulicht rein beispielhaft, aber keinesfalls beschränkend potentielle Therapiemöglichkeiten bzw. mögliche Indikationen für den Wirkstoff 3-Hydroxybuttersäure.

| **Indikation** | **Therapeutischer Effekt** |
|---|---|
| Schädel-Hirn-Trauma | Unter BHB sinkt die Apoptose und Nekrose-Rate von Nervenzellen. |
| Schlaganfall | Unter BHB sinkt die Apoptose und Nekrose-Rate von Nervenzellen. |
| Refeeding-Syndrom | Bei Anorexie, Absetzen enteraler oder parenteraler Ernährung und nach langen Hungerperioden kann der Konsum von Stärke oder Glucose zum Tod führen (siehe auch WHO-Schema Erdnusspaste). BHB kann hier als Therapeutikum zum schnelleren Erlangen einer normalen Nahrungsaufnahme eingesetzt werden. |
| Appetitzügler | BHB unterdrückt im Zentralnervensystem (ZNS) das Hungergefühl. |
| Epilepsie | Herkömmliche ketogene Diät zur signifikanten Reduzierung der Häufigkeit von Krampfanfällen hat extrem schlechte Patienten-Verträglichkeit. BHB bietet hier eine unmittelbar wirksame Alternative. |
| Morbus Alzheimer, Demenz | Unter BHB zeigen Patienten eine bessere kognitive Leistung. BHB ist auch für die Prävention von neurodegenerativen Erkrankungen wirksam. |
| Störungen der Fettsäureoxidation (z. B. Electron-Transfer-Protein Defekt) | Ausgleich eines Nährstoffmangels bei Defekt im Energiestoffwechsel. |

Daher ist es aus pharmazeutischer und klinischer Hinsicht wünschenswert, wirksame Präkursoren oder Metabolite auffinden zu können, welche physiologisch einen direkten oder indirekten Zugang zu 3-Hydroxybuttersäure oder deren Salzen ermöglichen, insbesondere im physiologischen Stoffwechsel des menschlichen oder tierischen Körpers.

Folglich hat es im Stand der Technik nicht an Versuchen gefehlt, physiologisch geeignete Präkursoren oder Metaboliten für 3-Hydroxybuttersäure bzw. deren Salze aufzufinden. Bislang wurden im Stand der Technik jedoch keine effizienten diesbezüglichen Verbindungen aufgefunden. Auch ist ein diesbezüglicher Zugang zu solchen Verbindungen nach dem Stand der Technik bislang nicht bzw. nicht ohne Weiteres möglich.

Die WO 2010/021766 A1 betrifft eine Verbindung, welche in Bezug auf (3R)-Hydroxybutyl-(3R)-Hydroxybutyrat enantiomer angereichertes 3-Hydroxybutyl-3-Hydroxybutyrat der Formel (I) ist, wobei die Verbindungen der Formel (I) ein wirksamer und schmackhafter Vorläufer des Ketokörpers (3R)-Hydroxybutyrat sind und zur Behandlung eines Zustands verwendet werden können, welcher durch einen erhöhten Plasmaspiegel an freien Fettsäuren bei einem Menschen oder Tier verursacht, verschlimmert oder damit verbunden ist (beispielsweise ein Zustand, bei welchem Gewichtsverlust oder Gewichtszunahme eine Rolle spielt) oder aber zur Förderung der Wachsamkeit oder zur Verbesserung der kognitiven Funktion oder zur Behandlung, Verhinderung oder Verringerung der Auswirkungen von Neurodegeneration, Toxizität durch freie Radikale, hypoxische Zustände oder Hyperglämie eingesetzt werden können.

Darüber hinaus betrifft die WO 2006/012490 A2 eine ketogene Verbindung mit der allgemeinen Formel (R(OCH(CH₃)CH₂C(O))ₙO)ₘ-A, wobei n eine ganze Zahl zwischen 1 und 10 bezeichnet, m eine ganze Zahl zwischen 1 und 200.000 bezeichnet, A ein Monosaccharid, Polysaccharid oder Oligosaccharidrest darstellt und R ausgewählt aus der Gruppe von H, C₁-C₆-Alkyl und Acetoacetyl ist.

Die WO 2004/108740 A2 betrifft Verbindungen und Zusammensetzungen, welche (R)-3-Hydroxybutyrat-Derivate enthalten, wobei die Verbindungen und Zusammensetzungen als Nahrungsergänzungsmittel zur Steigerung der körperlichen Leistungsfähigkeit und als Therapeutikum zur Linderung der Symptome von Erkrankungen, insbesondere neurologischen Erkrankungen wie Alzheimer oder ähnlichen Erkrankungen, verwendet werden, sowie deren Herstellungsverfahren, wobei in dem Verfahren beispielsweise ein überkritisches Lösungsmittel wie z. B. überkritisches Kohlendioxid verwendet wird und eine Lipase-katalysierte Veresterungs- oder Umesterungsreaktion durchgeführt wird, um die (R)-3-Hydroxybutyrat-Derivate herzustellen.

Weiterhin betrifft die WO 2013/150153 Ketokörper und Ketokörperester zur oralen Verabreichung zur Verbesserung oder zum Erhalt der Muskelkraft, wobei bestimmte Ester von Hydroxybutyratmonomeren organoleptisch akzeptabel sind und eine hohe Aufnahme vom Darm ins Blut aufweisen sollen, wodurch ein schneller Anstieg der Hydroxybutyratkonzentration im Blut und eine physiologische Reaktion einschließlich einer verbesserten Leistungsabgabe während des Trainings ermöglicht werden sollen, sowie Zusammensetzungen, welche diese Ketokörper oder Ketokörperester enthalten.

Die US 2018/303821 betrifft ein Verfahren zum Initiieren von Anästhesie, Sedierung und ein Verfahren zur Behandlung von Störungen, einschließlich Störungen des Zentralnervensystems, Störungen des peripheren Nervensystems, Depressionischämie und Behandlung mit einem Antikonvulsium durch Verabreichung einer wirksamen Menge einer Verbindung an einen Patienten.

Das der vorliegenden Erfindung zugrundeliegende Problem liegt also in der Bereitstellung eines effizienten Herstellungsverfahrens von physiologisch geeigneten bzw. physiologisch kompatiblen Präkursoren und/oder Metaboliten von 3-Hydroxybuttersäure (d. h. beta-Hydroxybuttersäure bzw. BHB bzw. 3-BHB) oder deren Salzen.

Ein solches Verfahren soll insbesondere die betreffenden BHB-Präkursoren und/oder BHB-Metaboliten in effizienter Weise zugänglich machen, insbesondere auch in größeren Mengen und ohne nennenswerte Mengen an toxischen Nebenprodukten.

In vollkommen überraschender Weise hat die Anmelderin nunmehr herausgefunden, dass Polyolester, nämlich Polyglycerinester, der 3-Hydroxybuttersäure (beta-Hydroxybuttersäure, BHB bzw. 3-BHB) einen effizienten und physiologisch wirksamen bzw. physiologisch kompatiblen Präkursor und/oder Metaboliten für den Ketokörper 3-Hydroxybuttersäure bzw. deren Salze darstellen, und hat in diesem Zusammenhang ein effizientes Herstellungsverfahren für diese Verbindungen auffinden bzw. entwickeln können, welches einen direkten und wirksamen, insbesondere ökonomischen wie auch großtechnisch umsetzbaren Zugang zu diesen Verbindungen ermöglicht.

Zur Lösung des zuvor geschilderten Problems schlägt die vorliegende Erfindung daher - gemäß einem **ersten** Aspekt der vorliegenden Erfindung - ein Verfahren zur Herstellung von Polyolestern, nämlich Polyglycerinestern, der 3-Hydroxybuttersäure (beta-Hydroxybuttersäure, BHB bzw. 3-BHB) gemäß Anspruch 1 vor; weitere, insbesondere besondere und/oder vorteilhafte Ausgestaltungen des erfindungsgemäßen Verfahrens sind Gegenstand der diesbezüglichen Verfahrensunteransprüche.

Weiterhin betrifft die vorliegende Erfindung - gemäß einem **zweiten** Aspekt der vorliegenden Erfindung - ein nach dem erfindungsgemäßen Verfahren erhältliches Reaktionsprodukt gemäß dem diesbezüglichen unabhängigen Anspruch (Anspruch 4) bzw. einen Polyolester, nämlich Polyglycerinester, der 3-Hydroxybuttersäure (beta-Hydroxybuttersäure, BHB bzw. 3-BHB) gemäß den diesbezüglichen Ansprüchen (Ansprüche 5 bis 10) bzw. ein diesbezüglich erhältliches Gemisch von zwei oder drei Polyolestern, nämlich Polyglycerinestern, der 3-Hydroxybuttersäure (beta-Hydroxybuttersäure, BHB bzw. 3-BHB), gemäß dem diesbezüglichen Anspruch (Anspruch 11).

Gleichermaßen betrifft die vorliegende Erfindung - gemäß einem **dritten** Aspekt der vorliegenden Erfindung - eine pharmazeutische Zusammensetzung, insbesondere Arzneimittel oder Medikament, gemäß dem diesbezüglichen unabhängigen Anspruch (Anspruch 12.

Darüber hinaus betrifft die vorliegende Erfindung - gemäß einem **vierten** Aspekt der vorliegenden Erfindung - ein erfindungsgemäßes Reaktionsprodukt bzw. einen erfindungsgemäßen Polyolester, nämlich Polyglycerinester, der 3-Hydroxybuttersäure (beta-Hydroxybuttersäure, BHB bzw. 3-BHB) bzw. ein erfindungsgemäßes Gemisch von mindestens zwei oder drei Polyolestern, nämlich Polyglycerinestern, der 3-Hydroxybuttersäure (beta-Hydroxybuttersäure, BHB bzw. 3-BHB) zur Verwendung bei der prophylaktischen und/oder therapeutischen Behandlung von Erkrankungen des menschlichen oder tierischen Körpers gemäß dem diesbezüglichen unabhängigen Anspruch (Anspruch 13).

Des Weiteren betrifft die vorliegende Erfindung - gemäß einem **fünften** Aspekt der vorliegenden Erfindung - ein Nahrungsmittel und/oder Lebensmittelerzeugnis gemäß dem diesbezüglichen unabhängigen Anspruch (Anspruch 14).

Schließlich betrifft die vorliegende Erfindung - gemäß einem **sechsten** Aspekt der vorliegenden Erfindung - die nicht therapeutische Verwendung eines erfindungsgemäßen Reaktionsprodukts bzw. eines erfindungsgemäßen Polyolesters, nämlich Polyglycerinesters, der 3-Hydroxybuttersäure (beta-Hydroxybuttersäure, BHB bzw. 3-BHB) bzw. eines erfindungsgemäßen Gemischs von mindestens zwei oder drei Polyolestern, nämlich Polyglycerinestern, der 3-Hydroxybuttersäure (beta-Hydroxybuttersäure, BHB bzw. 3-BHB) in einem Nahrungsmittel- und/oder Lebensmittelerzeugnis gemäß dem diesbezüglichen unabhängigen Anspruch (Anspruch 15).

Es versteht sich bei den nachfolgenden Ausführungen von selbst, dass Ausgestaltungen, Ausführungsformen, Vorteile und dergleichen, welche nachfolgend zu Zwecken der Vermeidung von Wiederholungen nur zu einem Erfindungsaspekt ausgeführt sind, selbstverständlich auch in Bezug auf die übrigen Erfindungsaspekte entsprechend gelten, ohne dass dies einer gesonderten Erwähnung bedarf.

Des Weiteren versteht es sich von selbst, dass einzelne Aspekte und Ausführungsformen der vorliegenden Erfindung auch in beliebiger Kombination mit anderen Aspekten und Ausführungsformen der vorliegenden Erfindung als offenbart gelten und insbesondere auch eine beliebige Kombination von Merkmalen und Ausführungsformen, wie sie sich aus den Rückbezügen aller Patentansprüche ergibt, umfangreich als offenbart gilt, und zwar im Hinblick auf alle sich ergebenden Kombinationsmöglichkeiten.

Bei allen nachstehend genannten relativen bzw. prozentualen gewichtsbezogenen Angaben, insbesondere relativen Mengen- oder Gewichtsangaben, ist weiterhin zu beachten, dass diese im Rahmen der vorliegenden Erfindung vom Fachmann derart auszuwählen sind, dass sie sich in der Summe unter Einbeziehung aller Komponenten bzw. Inhaltsstoffe, insbesondere wie nachfolgend definiert, stets zu 100 % bzw. 100 Gew.-% ergänzen bzw. addieren; dies versteht sich aber für den Fachmann von selbst.

Zudem gilt, dass alle im Folgenden genannten Werte- bzw. Parameterangaben oder dergleichen grundsätzlich mit genormten bzw. standardisierten oder explizit angegebenen Bestimmungsverfahren oder andernfalls mit dem Fachmann auf diesem Gebiet an sich geläufigen Bestimmungs- bzw. Messmethoden ermittelt bzw. bestimmt werden können.

Dies vorausgeschickt, wird die vorliegende Erfindung nunmehr nachfolgend im Detail erläutert.

Gegenstand der vorliegenden Erfindung - gemäß einem **ersten** Aspekt der vorliegenden Erfindung - ist somit ein Verfahren zur Herstellung von Polyolestern, nämlich Polyglycerinestern, der 3-Hydroxybuttersäure (beta-Hydroxybuttersäure, BHB bzw. 3-BHB),
wobei mindestens eine Verbindung der allgemeinen Formel (I)

   CH₃-CH(OH)-CH₂-C(O)OR¹ (I)
wobei in der allgemeinen Formel (I) der Rest R¹ Wasserstoff oder ein C₁-C₄-Alkyl, insbesondere ein C₁-C₄-Alkyl, bevorzugt Methyl oder Ethyl, besonders bevorzugt Ethyl, darstellt,
mit mindestens einem Polyglycerin der allgemeinen Formel (IIb)

   HO-CH₂-CH(OH)-CH₂-[O-CH₂-CH(OH)-CH₂]ₚ-OH (IIb)
wobei in der allgemeinen Formel (IIb) die Variable p eine ganze Zahl von 1 bis 4, insbesondere 1 oder 2, vorzugsweise 1, darstellt,
umgesetzt wird,
wobei die Umsetzung in Abwesenheit von Lösemitteln und/oder ohne jedwedes Lösemittel durchgeführt wird und
wobei die Umsetzung in Gegenwart eines Katalysators durchgeführt wird,
so dass als Reaktionsprodukt ein oder mehrere 3-Hydroxybuttersäure-Polyolester (III), nämlich 3-Hydroxybuttersäure-Polyglycerinester, erhalten werden,
wobei bei der Umsetzung gleichzeitig die Verbindung gemäß der allgemeinen Formel (IV)

   R¹-OH (IV)
gebildet wird, wobei in der allgemeinen Formel (IV) der Rest R¹ Wasserstoff oder ein C₁-C₄-Alkyl, insbesondere ein C₁-C₄-Alkyl, bevorzugt Methyl oder Ethyl, besonders bevorzugt Ethyl, darstellt, wobei die Verbindung gemäß der allgemeinen Formel (IV) der Umsetzung kontinuierlich entzogen wird.

Wie zuvor ausgeführt, hat die Anmelderin nämlich vollkommen überraschend herausgefunden, dass die auf diese Weise hergestellten Polyolester, nämlich Polyglycerinester, der 3-Hydroxybuttersäure (beta-Hydroxybuttersäure, BHB bzw. 3-BHB) - synonym auch als "3-Hydroxybuttersäure-Polyolester, nämlich 3-Hydroxybuttersäure-Polyglycerinester" bezeichnet (nachfolgend auch kurz nur als "BHB-Polyolester"/"3-BHB-Polyolester" bzw. "BHB-Polyglycerinester"/"3-BHB-Polyglycerinester" oder auch nur als "BHB-Ester"/"3-BHB-Ester" bezeichnet) - effiziente, da physiologisch verträgliche Präkursoren und/oder Metabolite der 3-Hydroxybuttersäure bzw. deren Salzen darstellen, welche pharmazeutisch bzw. klinisch auch in größeren Mengen zum Einsatz kommen können, da sie physiologisch kompatibel sind.

Die vorgenannten Polyolester, nämlich Polyglycerinester, der 3-Hydroxybuttersäure, welche durch das erfindungsgemäße Herstellungsverfahren erstmals in effizienter Weise zugänglich sind, stellen eine physiologisch und pharmakologisch relevante Alternative zu der freien 3-Hydroxybuttersäure bzw. deren Salzen dar.

Die Herstellung von Polyolestern, nämlich Polyglycerinestern, der 3-Hydroxybuttersäure mittels herkömmlicher organischer Synthese ist komplex und aufwendig, da 3-Hydroxybuttersäure verstärkt zur Polymerisation und anderen unerwünschten Nebenreaktionen (z. B. Wasserabspaltung, Zersetzung etc.) neigt. Im Rahmen der vorliegenden Erfindung konnte erstmals ein effizient arbeitendes Herstellungsverfahren bereitgestellt werden, mit welchem sich Polyolester, insbesondere Polyglycerinester, der 3-Hydroxybuttersäure ohne unerwünschte Nebenreaktionen herstellen lassen, insbesondere einstufig.

Das erfindungsgemäße Verfahren ermöglicht somit erstmalig die Bereitstellung untoxischer Polyolester, nämlich Polyglycerinester, der 3-Hydroxybuttersäure aus an sich bekannten, kommerziell verfügbaren und vor allem physiologisch unbedenklichen Komponenten bzw. Edukten (Ausgangsverbindungen). Die resultierenden Polyolester, insbesondere Polyglycerinester, der 3-Hydroxybuttersäure, können physiologisch, insbesondere im Magen und/oder im Darm, aufgespalten werden und das Zielmolekül "3-Hydroxybuttersäure" bzw. deren Salze als Wirkstoff bzw. Wirkkomponente freisetzen bzw. generieren.

Darüber hinaus weisen die vorgenannten Polyolester, nämlich Polyglycerinester, der 3-Hydroxybuttersäure auch einen akzeptablen Geschmack auf, um eine Kompatibilität auch bei oraler Verabreichung größerer Mengen über einen längeren Zeitraum zu gewährleisten (z. B. Verabreichung von 50 g Tagesdosis oder mehr).

Gleichermaßen ermöglicht es das erfindungsgemäße Herstellungsverfahren die Polyolester, nämlich Polyglycerinester, der 3-Hydroxybuttersäure frei von toxischen Verunreinigungen bereitzustellen.

Darüber hinaus kann bei entsprechenden Ausgangsmaterialien die Herstellung auch enantioselektiv durchgeführt werden. So ermöglicht es beispielsweise das erfindungsgemäße Herstellungsverfahren, die biologisch relevante Form, d. h. das (R)-Enantiomer anzureichern, insbesondere durch Enzymkatalyse, um bei oraler Verabreichung das renale System von Patienten nicht zu belasten (d. h. Elimination über die Nieren). Grundsätzlich ist es aber auch möglich und kann es unter bestimmten Voraussetzungen zweckdienlich sein, das (S)-Enantiomer anzureichern.

Darüber hinaus ist das erfindungsgemäße Herstellungsverfahren, einschließlich optionaler Weiterverarbeitungs- bzw. Aufreinigungsverfahrensschritte, wirtschaftlich bzw. ökonomisch betreibbar und auch großtechnisch umsetzbar.

Insbesondere verwendet das erfindungsgemäße Herstellungsverfahren kommerziell verfügbare Edukte und ermöglicht darüber hinaus eine relativ einfache Verfahrensführung auch bei großtechnischer Umsetzung.

Im Gegensatz zu herkömmlichen Herstellungsverfahren des Standes der Technik kommt das erfindungsgemäße Herstellungsverfahren ohne komplexe Edukte aus und verläuft nur einstufig. Dennoch werden im Rahmen des erfindungsgemäßen Herstellungsverfahrens exzellente Ausbeuten erzielt, wobei in gleicher Weise die Bildung von Nebenprodukten minimiert bzw. vermieden wird.

Darüber hinaus ist das erfindungsgemäßen Verfahren einfach und wirtschaftlich. Insbesondere wird das erfindungsgemäße Verfahren in Abwesenheit von Lösemitteln und/oder ohne jedwedes Lösemittel durchgeführt (d. h. also als Reaktion in Masse bzw. als Reaktion in Substanz bzw. als sogenannte *Bulk Reaction*); folglich sind die erhaltenen Reaktionsprodukte nicht mit Lösemittel verunreinigt und es muss kein Lösemittel nach Durchführung des Verfahrens bzw. der Reaktion aufwendig und energieträchtig entfernt und entsorgt bzw. rezykliert werden. Es werden zudem auch keine toxischen Nebenprodukte gebildet.

Das erfindungsgemäße Herstellungsverfahren führt üblicherweise zu einem Gemisch verschiedener Polyolester, nämlich Polyglycerinester, der 3-Hydroxybuttersäure, d. h. zu einem Gemisch von mindestens zwei, insbesondere mindestens drei voneinander verschiedenen Polyolestern, nämlich Polyglycerinestern, der 3-Hydroxybuttersäure. Das resultierende Rohreaktionsprodukt bzw. Rohgemisch kann mit an sich bekannten Methoden aufgereinigt werden, insbesondere von gegebenenfalls noch vorhandenen Edukten und/oder gegebenenfalls vorhandenen Nebenprodukten befreit werden, und darüber hinaus - sofern gewünscht - mit ebenfalls an sich bekannten Methoden aufgespalten werden, insbesondere destillativ und/oder chromatographisch (z. B. Fraktionierung in die einzelnen Polyolester, d. h. Mono-, Di-, Tri- usw. Polyolester der 3-Hydroxybuttersäure, oder aber Fraktionierung in Fraktionen mit angereichertem und abgereichertem Anteil einzelner etc.).

Gemäß einer besonderen Ausführungsform der vorliegenden Erfindung kann die Verbindung der allgemeinen Formel (I) entweder in racemischer Form oder aber in Form des (R)-Enantiomers eingesetzt werden. Die (R)-Konfiguration bezieht sich auf das chirale Kohlenstoffatom in 3-Position der Verbindung der allgemeinen Formel (I).

Erfindungsgemäß bevorzugt ist es, wenn in der allgemeinen Formel (I) der Rest R¹ Ethyl darstellt.

Mit anderen Worten ist es erfindungsgemäß bevorzugt, dass als Verbindung der allgemeinen Formel (I) 3-Hydroxybuttersäureethylester (Ethyl-3-hydroxybutyrat) der Formel CH₃-CH(OH)-CH₂-C(O)OC₂H₅ eingesetzt wird.

Dies ermöglicht eine besonders effiziente Verfahrensführung und hohe Ausbeuten mit minimierter bzw. unterdrückter Nebenproduktbildung. Zudem ist der 3-Hydroxybuttersäureethylester auch in großen Mengen kommerziell verfügbar und zudem ökonomisch effizienter als die freie Säure (d. h. 3-Hydroxybuttersäure) umsetzbar. Insbesondere kann der 3-Hydroxybuttersäureethylester als Ausgangsverbindung großtechnisch z. B. durch Claisen-Kondensation von Ethylacetat gewonnen werden.

Bei dem erfindungsgemäßen Verfahren wird die Umsetzung in Abwesenheit von Lösemitteln und/oder ohne jedwedes Lösemittel durchgeführt. D. h. die Umsetzung wird also als Reaktion in Masse bzw. als Reaktion in Substanz bzw. als sogenannte *Bulk Reaction* durchgeführt. Dies hat den Vorteil, dass die erhaltenen Reaktionsprodukte nicht mit Lösemittel verunreinigt sind und kein Lösemittel nach Durchführung des Verfahrens bzw. der Reaktion aufwendig und energieträchtig entfernt und entsorgt bzw. rezykliert werden muss. Überraschenderweise verläuft das Verfahren bzw. die Reaktion dennoch mit hohen Umsätzen und Ausbeuten und zumindest im Wesentlichen ohne signifikante Nebenproduktbildung.

Gemäß der vorliegenden Erfindung wird die Umsetzung in Gegenwart eines Katalysators, insbesondere eines Enzyms und/oder eines metallhaltigen und/oder metallbasierten sauren oder basischen Katalysators, vorzugsweise in Gegenwart eines Enzyms, durchgeführt. Bei dieser besonderen Ausführungsform ist es bevorzugt, wenn der Katalysator nach der Umsetzung rezykliert wird.

Wie zuvor ausgeführt, kann gemäß einer besonderen Ausführungsform des erfindungsgemäßen Herstellungsverfahrens die Umsetzung in Gegenwart eines Enzyms als Katalysator durchgeführt werden.

Dabei kann das Enzym insbesondere ausgewählt sein aus Synthetasen (Ligasen), Katalasen, Esterasen, Lipasen und deren Kombinationen. Erfindungsgemäß werden als Synthetasen (synonym Ligasen) insbesondere Enzyme aus der Klasse der Ligasen bezeichnet; Ligasen sind Enzyme, welche das Verknüpfen zweier oder mehrerer Moleküle durch eine kovalente Bindung katalysieren. Katalasen im Sinne der vorliegenden Erfindung sind insbesondere Enzyme, welche Wasserstoffperoxid zu Sauerstoff und Wasser umzusetzen imstande sind. Der Begriff der Esterasen bezeichnet insbesondere Enzyme, welche imstande sind, Ester hydrolytisch in Alkohol und Säure aufzuspalten (Verseifung); es handelt sich somit insbesondere um Hydrolasen, wobei fettspaltende Esterasen auch als Lipasen bezeichnet werden. Lipasen im Sinne der vorliegenden Erfindung sind insbesondere Enzyme, welche von Lipiden, wie Glyceriden, freie Fettsäuren abzuspalten imstande sind (Lipolyse).

Im Rahmen der vorliegenden Erfindung kann sich das als Katalysator eingesetzte Enzym insbesondere ableiten von *Candida antarctica, Mucor miehei (Rhizomucor miehei), Thermomyces lanuginosus, Candida rugosa, Aspergillus oryzae, Pseudomonas cepacia, Pseudomonas fluorescens, Rhizopus delemar* und *Pseudomonas* sp. sowie deren Kombinationen, vorzugsweise von *Candida antarctica, Mucor miehei (Rhizomucor miehei)* und *Thermomyces lanuginosus.*

Gemäß einer besonderen Ausführungsform kann das Enzym in immobilisierter Form, immobilisiert auf einem Träger, vorzugsweise auf einem polymeren Träger, bevorzugt auf einem polymeren organischen Träger, besonders bevorzugt mit hydrophoben Eigenschaften, ganz besonders bevorzugt auf einem poly(meth)acrylharzbasierten Träger, eingesetzt werden.

Wie zuvor im Zusammenhang mit der Verwendung eines Katalysators im Allgemeinen dargelegt, ist es bevorzugt, im Fall der Verwendung eines Enzyms als Katalysator das Enzym nach der Umsetzung zu rezyklieren.

Sofern die Umsetzung im Rahmen des erfindungsgemäßen Herstellungsverfahrens in Gegenwart eines Enzyms als Katalysator durchgeführt wird, ist es bevorzugt, wenn die Umsetzung bei Temperaturen im Bereich von 10 °C bis 80 °C, insbesondere im Bereich von 20 °C bis 80 °C, vorzugsweise im Bereich von 25 °C bis 75 °C, besonders bevorzugt im Bereich von 45 °C bis 75 °C, ganz besonders bevorzugt im Bereich von 50 °C bis 70 °C, durchgeführt wird.

Im Fall der Verwendung eines Enzyms als Katalysator kann die Menge des eingesetzten Enzyms in weiten Bereichen variieren. Insbesondere kann das Enzym in Mengen, bezogen auf die Gesamtmenge der Ausgangsverbindungen (I) und (II), im Bereich von 0,001 Gew.-% bis 20 Gew.-%, insbesondere im Bereich von 0,01 Gew.-% bis 15 Gew.-%, vorzugsweise im Bereich von 0,1 Gew.-% bis 15 Gew.-%, bevorzugt im Bereich von 0,5 Gew.-% bis 10 Gew.-%, eingesetzt werden. Dennoch kann es einzelfallbedingt oder anwendungsbezogen erforderlich sein, von den vorgenannten Mengen abzuweichen, ohne dass der Rahmen der vorliegenden Erfindung verlassen ist.

Wenn gemäß einer besonderen Ausführungsform der vorliegenden Erfindung die Umsetzung in Gegenwart eines Enzyms als Katalysator durchgeführt wird, kann auch der angewendete Druckbereich in weiten Bereichen variieren. Insbesondere kann bei Umsetzung in Gegenwart eines Enzyms als Katalysator die Umsetzung bei einem Druck im Bereich von 0,0001 bar bis 10 bar, insbesondere im Bereich von 0,001 bar bis 5 bar, vorzugsweise im Bereich von 0,01 bar bis 2 bar, besonders bevorzugt im Bereich von 0,05 bar bis 1 bar, ganz besonders bei etwa 1 bar, durchgeführt werden.

Gemäß einer alternativen Ausführungsform der vorliegenden Erfindung kann die Umsetzung in Gegenwart eines metallhaltigen und/oder metallbasierten, sauren oder basischen Katalysators durchgeführt werden.

Gemäß dieser alternativen Ausführungsform der vorliegenden Erfindung, wonach die Umsetzung in Gegenwart eines metallhaltigen und/oder metallbasierten, sauren oder basischen Katalysators durchgeführt wird, kann der Katalysator insbesondere ausgewählt sein aus (i) basischen Katalysatoren, insbesondere Alkali- oder Erdalkalihydroxyden und Alkali- oder Erdalkalialkoholaten, wie NaOH, KOH, LiOH, Ca(OH)₂, NaOMe, KOMe und Na(OBu-tert.), (ii) sauren Katalysatoren, insbesondere Mineralsäuren, und organischen Säuren, wie Schwefelsäure, Salzsäure, Phosphorsäure, Salpetersäure, Sulfonsäuren, Methansulfonsäure, para-Toluolsulfonsäure und Carbonsäuren, (iii) Lewis-Säuren, insbesondere Lewis-Säuren auf der Basis von Titan-, Zinn-, Zink- und Aluminiumverbindungen, wie Titantetrabutylat, Zinnsäuren, Zinkacetat, Aluminiumtrichlorid und Aluminiumtriisopropyl und (iv) heterogenen Katalysatoren, insbesondere auf der Basis von mineralischen Silikaten, Germanaten, Carbonaten und Aluminiumoxiden, wie Zeolithen, Montmorilloniten, Mordeniten, Hydrotalciten und Tonerden, sowie deren Kombinationen.

Bei dieser Ausführungsform kann als Katalysator insbesondere ein Alkali- oder Erdalkalialkoholat eingesetzt werden.

Insbesondere ist es auch bei dieser Ausführungsform bevorzugt, wenn der Katalysator auf Basis des metallhaltigen und/oder metallbasierten sauren oder basischen Katalysators nach der Umsetzung rezykliert wird.

Wenn gemäß der besonderen Ausführungsform der vorliegenden Erfindung die Umsetzung in Gegenwart eines metallhaltigen und/oder metallbasierten, sauren oder basischen Katalysators erfolgt, können die Temperaturen in weiten Bereichen variiert werden. Insbesondere kann die Umsetzung in Gegenwart eines metallhaltigen und/oder metallbasierten sauren oder basischen Katalysators bei Temperaturen im Bereich von 20 °C bis 150°C, insbesondere im Bereich von 50 °C bis 140 °C, vorzugsweise im Bereich von 70 °C bis 130 °C, besonders bevorzugt im Bereich von 80 °C bis 125 °C, ganz besonders bevorzugt im Bereich von 100 °C bis 120 °C, durchgeführt werden.

Weiterhin kann auch bei dieser Ausführungsform der Katalysator (d. h. der metallhaltige und/oder metallbasierte, saure oder basische Katalysator) in weiten Mengenbereichen variiert werden: So kann der Katalysator auf Basis eines metallhaltigen und/oder metallbasierten, sauren oder basischen Katalysators in Mengen, bezogen auf die Gesamtmenge der Ausgangsverbindungen (I) und (II), im Bereich von 0,01 Gew.-% bis 30 Gew.-%, insbesondere im Bereich von 0,05 Gew.-% bis 15 Gew.-%, vorzugsweise im Bereich von 0,1 Gew.-% bis 15 Gew.-%, bevorzugt im Bereich von 0,2 Gew.-% bis 10 Gew.-%, eingesetzt werden. Dennoch ist es anwendungsbezogen oder einzelfallbedingt möglich, von den vorgenannten Mengen abzuweichen, ohne dass der Rahmen der vorliegenden Erfindung verlassen ist.

Wenn gemäß dieser besonderen Ausführungsform der vorliegenden Erfindung die Umsetzung in Gegenwart eines metallhaltigen und/oder metallbasierten, sauren oder basischen Katalysators erfolgt, kann der Druckbereich gleichermaßen in einem weiten Regime variieren: Insbesondere kann die Umsetzung in Gegenwart eines metallhaltigen und/oder metallbasierten, sauren oder basischen Katalysators bei einem Druck im Bereich von 0,0001 bar bis 10 bar, insbesondere im Bereich von 0,001 bar bis 5 bar, vorzugsweise im Bereich von 0,01 bar bis 2 bar, besonders bevorzugt im Bereich von 0,05 bar bis 1 bar, ganz besonders bei etwa 1 bar, durchgeführt werden.

Was die Menge an Edukten bzw. Ausgangsverbindungen anbelangt, so kann auch diese in weiten Bereichen variiert werden.

Unter Berücksichtigung von Verfahrensökonomie und Optimierung des Verfahrensablaufs, insbesondere im Hinblick auf die Minimierung von Nebenprodukten, ist es vorteilhaft, wenn die Verbindung der allgemeinen Formel (I), bezogen auf die Hydroxylgruppen des Polyols (II), insbesondere Polyglycerins, in molaren Mengen in einem Bereich von äquimolarer Menge bis zu einem molaren Überschuss von 200 Mol-%, insbesondere in einem Bereich von äquimolarer Menge bis zu einem molaren Überschuss von 150 Mol-%, vorzugsweise in einem Bereich von äquimolarer Menge bis zu einem molaren Überschuss von 100 Mol-%, eingesetzt wird.

Gleichermaßen unter Berücksichtigung von Verfahrensökonomie und Optimierung des Verfahrensablaufs, insbesondere im Hinblick auf die Minimierung von Nebenprodukten, ist es vorteilhaft, wenn die Verbindung der allgemeinen Formel (I) und das Polyol (II), insbesondere Polyglycerin, in einem Molverhältnis von Verbindung der allgemeinen Formel (I) / Polyol (II) in einem Bereich von 1 :1 bis 10 : 1, insbesondere in einem Bereich von 2 : 1 bis 8 : 1, vorzugsweise in einem Bereich von 3 : 1 bis 6 : 1, eingesetzt werden.

Gemäß dem erfindungsgemäßen Verfahren entspricht Polyglycerinen der allgemeinen Formel (IIb)

HO-CH₂-CH(OH)-CH₂-[O-CH₂-CH(OH)-CH₂]ₚ-OH (IIb)

wobei in der allgemeinen Formel (IIb) die Variable p eine ganze Zahl von 1 bis 4, insbesondere 1 oder 2, vorzugsweise 1, darstellt.

Gemäß einer weiteren besonderen Ausführungsform des erfindungsgemäßen Verfahrens kann das Polyol (II) ein Diglycerin der Formel (IIc)

HO-CH₂-CH(OH)-CH₂-O-CH₂-CH(OH)-CH₂-OH (IIc)

sein.

Gemäß einer bevorzugten Ausführungsform der vorliegenden Erfindung betrifft die vorliegende Erfindung gemäß diesem Erfindungsaspekt ein Verfahren zur Herstellung von Polyolestern, nämlich Polyglycerinestern, der 3-Hydroxybuttersäure (beta-Hydroxybuttersäure, BHB bzw. 3-BHB), insbesondere ein wie zuvor beschriebenes Verfahren,
wobei mindestens eine Verbindung der allgemeinen Formel (I)

   CH₃-CH(OH)-CH₂-C(O)OR¹ (I)
wobei in der allgemeinen Formel (I) der Rest R¹ Wasserstoff oder ein C₁-C₄-Alkyl, insbesondere ein C₁-C₄-Alkyl, bevorzugt Methyl oder Ethyl, besonders bevorzugt Ethyl, darstellt,
mit mindestens einem Polyglycerinen der allgemeinen Formel (IIb)

   HO-CH₂-CH(OH)-CH₂-[O-CH₂-CH(OH)-CH₂]ₚ-OH (IIb)
wobei in der allgemeinen Formel (IIb) die Variable p eine ganze Zahl von 1 bis 4, insbesondere 1 oder 2, vorzugsweise 1, darstellt,
so dass als Reaktionsprodukt ein oder mehrere 3-Hydroxybuttersäure-Polyolester (III), nämlich 3-Hydroxybuttersäure-Polyglycerinester, erhalten werden.

Gemäß einer weiteren bevorzugten Ausführungsform der vorliegenden Erfindung betrifft die vorliegende Erfindung gemäß diesem Erfindungsaspekt ein Verfahren zur Herstellung von Polyolestern, nämlich Polyglycerinestern, der 3-Hydroxybuttersäure (beta-Hydroxybuttersäure, BHB bzw. 3-BHB), insbesondere ein wie zuvor beschriebenes Verfahren,
wobei mindestens eine Verbindung der allgemeinen Formel (I)

   CH₃-CH(OH)-CH₂-C(O)OR¹ (I)
wobei in der allgemeinen Formel (I) der Rest R¹ Ethyl darstellt,
mit mindestens Polyglycerinen der allgemeinen Formel (IIb)

   HO-CH₂-CH(OH)-CH₂-[O-CH₂-CH(OH)-CH₂]ₚ-OH (IIb)
wobei in der allgemeinen Formel (IIb) die Variable p eine ganze Zahl 1 oder 2, vorzugsweise 1, darstellt,
so dass als Reaktionsprodukt ein oder mehrere 3-Hydroxybuttersäure-Polyolester (III), nämlich 3-Hydroxybuttersäure-Polyglycerinester, erhalten werden.

Eine erfindungsgemäß besonders bevorzugte Vorgehensweise wird durch das nachfolgende Reaktions- bzw. Syntheseschema veranschaulicht (wobei in Abhängigkeit von der Reaktionsführung entweder einzelne der Ester oder ein Gemisch von zwei oder mehreren hiervon erhalten werden):

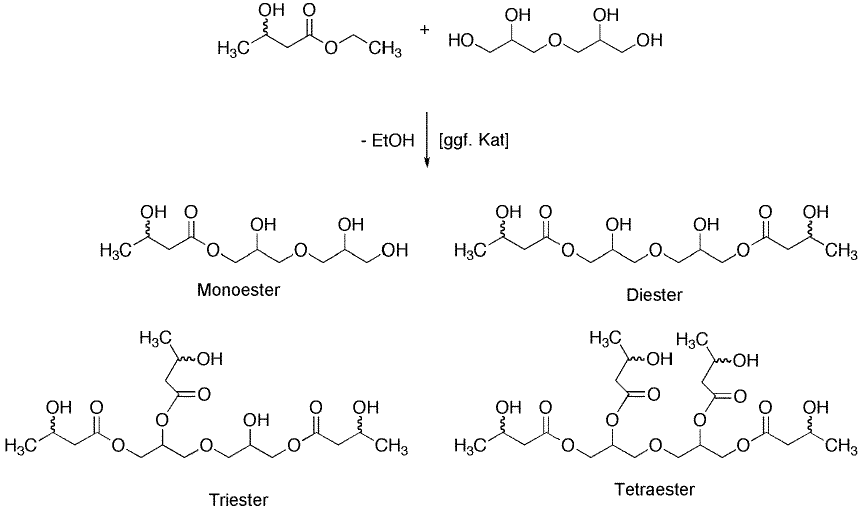

Im Rahmen des erfindungsgemäßen Verfahrens wird bei der Umsetzung gleichzeitig die Verbindung gemäß der allgemeinen Formel (IV)

R¹-OH (IV)

gebildet, wobei in der allgemeinen Formel (IV) der Rest R¹ Wasserstoff oder ein C₁-C₄-Alkyl, insbesondere ein C₁-C₄-Alkyl, bevorzugt Methyl oder Ethyl, besonders bevorzugt Ethyl, darstellt.

In diesem Zusammenhang ist es vorgesehen, dass die Verbindung gemäß der allgemeinen Formel (IV) der Umsetzung kontinuierlich entzogen wird, insbesondere mittels vorzugsweise kontinuierlicher destillativer Entfernung. Auf diese Weise wird das Reaktionsgleichgewicht in effizienter Weise auf die Seite der Reaktionsprodukte verlagert. Auch wird auf diese Weise die Bildung von Nebenprodukten minimiert bzw. verhindert.

Im Rahmen des erfindungsgemäßen Herstellungsverfahrens kann das Reaktionsprodukt, insbesondere die Zusammensetzung des Reaktionsprodukts, insbesondere das Vorhandensein verschiedener 3-Hydroxybuttersäure-Polyolester (III), nämlich 3-Hydroxybuttersäure-Polyglycerinester, und deren Anteil im Fall eines Gemischs, mittels der Umsetzungsbedingungen kontrolliert und/oder gesteuert werden, insbesondere durch Auswahl der Umsetzungstemperatur (Reaktionstemperatur) und/oder Auswahl des Umsetzungsdrucks (Reaktionsdrucks) und/oder Vorsehen eines Katalysators und dessen Auswahl in Bezug auf Art und/oder Menge und/oder Auswahl der Mengen der Ausgangsverbindungen (Edukte) und/oder Vorsehen der Entfernung der Verbindung gemäß der allgemeinen Formel (IV), wie zuvor definiert.

Im Anschluss an die Umsetzung kann das erhaltene Reaktionsprodukt weiteren üblichen bzw. an sich bekannten Aufreinigungs- bzw. Aufarbeitungsschritten unterzogen werden.

In diesem Zusammenhang kann das erhaltene Reaktionsprodukt nach erfolgter Umsetzung fraktioniert werden, insbesondere destillativ fraktioniert werden.

Auch können nichtumgesetzte Ausgangsverbindungen (I) und/oder (II) aus dem Reaktionsprodukt abgetrennt und anschließend rezykliert werden.

Gemäß einer besonderen Ausführungsform des erfindungsgemäßen Herstellungsverfahrens kann insbesondere derart vorgegangen werden, dass im Reaktionsprodukt nach erfolgter Umsetzung noch vorhandene Hydroxylgruppen zumindest teilweise, vorzugsweise vollständig, funktionalisiert, insbesondere verestert werden. Insbesondere kann sich der Umsetzung eine teilweise, insbesondere vollständige Funktionalisierung, insbesondere Veresterung, noch vorhandener Hydroxylgruppen anschließen.

Bei dieser besonderen Ausführungsform des erfindungsgemäßen Verfahrens kann insbesondere die Funktionalisierung, insbesondere Veresterung, der Hydroxylgruppen durch Reaktion mit einem Carbonsäureanhydrid, insbesondere C₂-C₃₀-Carbonsäureanhydrid, bevorzugt C₂-C₁₀-Carbonsäureanhydrid, vorzugsweise C₇-Carbonsäureanhydrid, erfolgen. Bei dem C₂-C₃₀-Carbonsäureanhydrid, bevorzugt C₂-C₁₀-Carbonsäureanhydrid, kann es sich um ein lineares (d. h. geradkettiges) oder verzweigtes, gesättigtes oder ein- oder mehrfach ungesättigtes C₂-C₃₀-Carbonsäureanhydrid, bevorzugt C₂-C₁₀-Carbonsäureanhydrid, handeln.

Eine erfindungsgemäß besonders bevorzugte Vorgehensweise, welche eine Funktionalisierung, insbesondere Veresterung, noch vorhandener Hydroxylgruppen im Anschluss an die Umsetzung vorsieht, wird durch das nachfolgende Reaktions- bzw. Syntheseschema veranschaulicht (wobei in Abhängigkeit von der Reaktionsführung bei der Umsetzung entweder einzelne der Ester oder ein Gemisch von zwei oder mehreren hiervon erhalten werden und wobei in dem nachfolgenden Reaktions- bzw. Syntheseschema der Rest R einen Rest der Formel CH₃ - (CH₂)_{x = 0-28} - C(O) - bezeichnet): 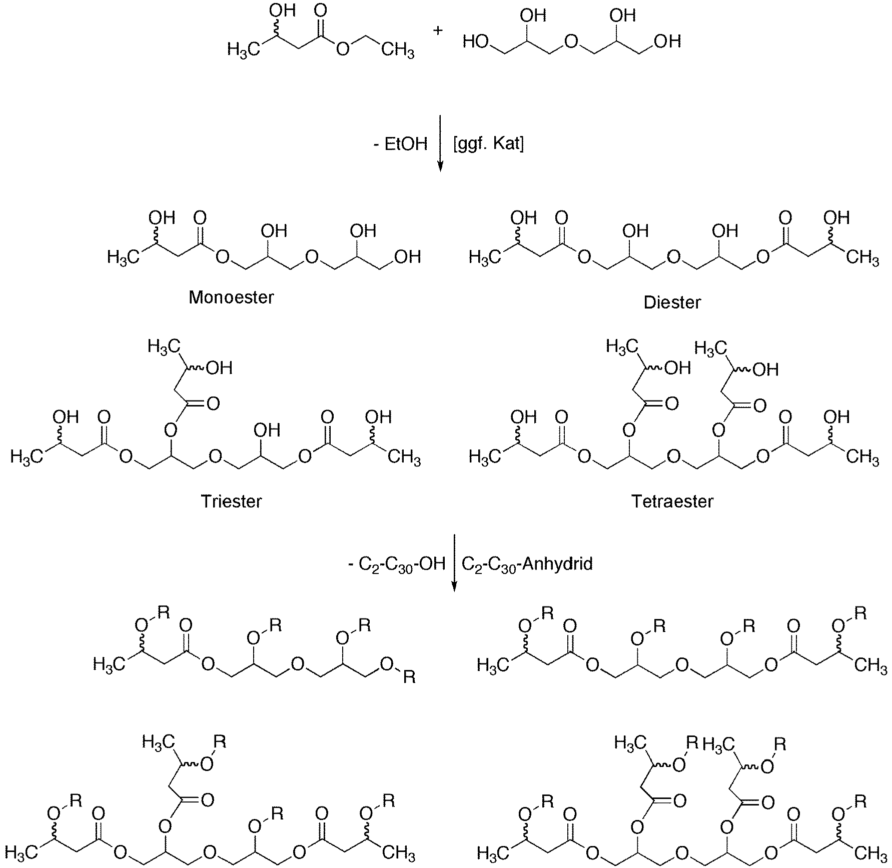 Insbesondere werden bei dem erfindungsgemäßen Herstellungsverfahren als Reaktionsprodukt ein oder mehrere 3-Hydroxybuttersäure-Polyolester (III), nämlich 3-Hydroxybuttersäure-Polyglycerinester, der allgemeinen Formel (IIIb)

R²O-CH₂-CH(OR²)-CH₂-[O-CH₂-CH(OR²)-CH₂]ₚ-OR² (IIIb)

erhalten,
wobei in der allgemeinen Formel (IIIb)
   - die Variable p eine ganze Zahl von 1 bis 4, insbesondere 1 oder 2, vorzugsweise 1, darstellt,
   - R², unabhängig voneinander, darstellt: Wasserstoff, einen Rest CH₃-CH(OH)-CH₂-C(O)- oder einen Rest CH₃-CH(OR³)-CH₂-C(O)- mit R³ = (C₁-C₂₉-Alkyl) - C(O) - oder einen Rest (C₁-C₂₉-Alkyl) - C(O) - , jedoch mit der Maßgabe, dass mindestens ein Rest R², insbesondere mindestens zwei Reste R², keinen Wasserstoff darstellt, und mit der Maßgabe, dass mindestens ein Rest R², insbesondere mindestens zwei Reste R², einen Rest CH₃- CH(OH) - CH₂-C(O)- oder einen Rest CH₃-CH(OR³)-CH₂-C(O)- mit R³ = (C₁-C₂₉-Alkyl) - C(O) - darstellt.

Insbesondere kann in der vorstehenden allgemeinen Formel (IIIb) R², unabhängig voneinander, Wasserstoff oder einen Rest CH₃- CH(OH) - CH₂- C(O) - darstellen, jedoch mit der Maßgabe, dass mindestens ein Rest R², insbesondere mindestens zwei Reste R², keinen Wasserstoff darstellt.

Insbesondere kann in der allgemeinen Formel (IIIb) R², unabhängig voneinander, darstellen: einen Rest CH₃-CH(OH)-CH₂-C(O)- oder einen Rest CH₃-CH(OR³)-CH₂-C(O)-mitR³ = (C₁-C₂₉-Alkyl) - C(O) - oder einen Rest (C₁-C₂₉-Alkyl) - C(O) - , jedoch mit der Maßgabe, dass mindestens ein Rest R², insbesondere mindestens zwei Reste R², einen Rest CH₃- CH(OH)-CH₂-C(O) - oder einen Rest CH₃-CH(OR³)-CH₂-C(O)- mit R³ = (C₁-C₂₉-Alkyl) - C(O) - darstellt.

Insbesondere kann in der allgemeinen Formel (IIIb) der C₁-C₂₉-Alkyl-Rest ein linearer (geradkettiger) oder verzweigter, gesättigter oder ein- oder mehrfach ungesättigter C₁-C₂₉-Alkylrest sein; bevorzugt kann der C₁-C₂₉-Alkyl-Rest ein C₁-C₉-Alkyl-Rest sein (insbesondere ein linearer oder verzweigter, gesättigter oder ein- oder mehrfach ungesättigter C₁-C₉-Alkylrest).

Gemäß einer besonderen Ausführungsform des erfindungsgemäßen Verfahrens können als Reaktionsprodukt insbesondere ein oder mehrere 3-Hydroxybuttersäure-Polyolester (III), nämlich 3-Hydroxybuttersäure-Polyglycerinester, der allgemeinen Formel (IIIc)

R²O-CH₂-CH(OR²)-CH₂-O-CH₂-CH(OR²)-CH₂-OR² (IIIc)

erhalten werden,
wobei in der allgemeinen Formel (IIIc) R², unabhängig voneinander, darstellt: Wasserstoff, einen Rest CH₃-CH(OH)-CH₂-C(O)- oder einen Rest CH₃-CH(OR³)-CH₂-C(O)- mit R³ = (C₁-C₂₉-Alkyl) - C(O) - oder einen Rest (C₁-C₂₉-Alkyl) - C(O) - , jedoch mit der Maßgabe, dass mindestens ein Rest R², insbesondere mindestens zwei Reste R², keinen Wasserstoff darstellt, und mit der Maßgabe, dass mindestens ein Rest R², insbesondere mindestens zwei Reste R², einen Rest CH₃-CH(OH)-CH₂-C(O)- oder einen Rest CH₃-CH(OR³)-CH₂-C(O) - mit R³ = (C₁-C₂₉-Alkyl) - C(O) - darstellt.

Insbesondere kann in der vorstehenden allgemeinen Formel (IIIc) R², unabhängig voneinander, Wasserstoff oder einen Rest CH₃-CH(OH)-CH₂-C(O)-darstellen, jedoch mit der Maßgabe, dass mindestens ein Rest R², insbesondere mindestens zwei Reste R², keinen Wasserstoff darstellt.

Insbesondere kann in der vorstehenden allgemeinen Formel (IIIc) R², unabhängig voneinander, darstellen: einen Rest CH₃-CH(OH)-CH₂-C(O)- oder einen Rest CH₃-CH(OR³)-CH₂-C(O)- mit R³ = (C₁-C₂₉-Alkyl) - C(O) - oder einen Rest (C₁-C₂₉-Alkyl) - C(O) - , jedoch mit der Maßgabe, dass mindestens ein Rest R², insbesondere mindestens zwei Reste R², einen Rest CH₃-CH(OH)-CH₂-C(O) - oder einen Rest CH₃-CH(OR³)-CH₂-C(O)- mit R³ = (C₁-C₂₉-Alkyl) - C(O) - darstellt.

Insbesondere kann in der allgemeinen Formel (IIIc) der C₁-C₂₉-Alkyl-Rest ein linearer (geradkettiger) oder verzweigter, gesättigter oder ein- oder mehrfach ungesättigter C₁-C₂₉-Alkylrest sein; bevorzugt kann der C₁-C₂₉-Alkyl-Rest ein C₁-C₉-Alkyl-Rest sein (insbesondere ein linearer oder verzweigter, gesättigter oder ein- oder mehrfach ungesättigter C₁-C₉-Alkylrest).

Gemäß einer weiteren besonderen Ausführungsform des erfindungsgemäßen Verfahrens kann als Reaktionsprodukt ein Gemisch von mindestens zwei voneinander verschiedenen 3-Hydroxybuttersäure-Polyolestern (III), nämlich 3-Hydroxybuttersäure-Polyglycerinestern, insbesondere wie zuvor definiert, erhalten werden.

Gemäß einer weiteren besonderen Ausführungsform des erfindungsgemäßen Verfahrens kann als Reaktionsprodukt ein Gemisch von mindestens drei voneinander verschiedenen 3-Hydroxybuttersäure-Polyolestern (III), nämlich 3-Hydroxybuttersäure-Polyglycerinestern, insbesondere wie zuvor definiert, erhalten werden.

Wie zuvor bereits ausgeführt, wird das erfindungsgemäße Verfahren in Abwesenheit von Lösemitteln und/oder ohne jedwedes Lösemittel durchgeführt (d. h. also als Reaktion in Masse bzw. als Reaktion in Substanz bzw. als sogenannte *Bulk Reaction).* Dies hat den Vorteil, dass die erhaltenen Reaktionsprodukte nicht mit Lösemittel verunreinigt sind und kein Lösemittel nach Durchführung des Verfahrens bzw. der Reaktion aufwendig und energieträchtig entfernt und entsorgt bzw. rezykliert werden muss. Überraschenderweise verläuft das Verfahren bzw. die Reaktion dennoch mit hohen Umsätzen und Ausbeuten und zumindest im Wesentlichen ohne signifikante Nebenproduktbildung.

Weiterer Gegenstand - gemäß einem **zweiten** Aspekt der vorliegenden Erfindung - ist das nach dem erfindungsgemäßen Verfahren erhältliche bzw. erfindungsgemäße Reaktionsprodukt (d. h. ein oder mehrere 3-Hydroxybuttersäure-Polyolester, nämlich 3-Hydroxybuttersäure-Polyglycerinester, oder deren Gemische).

Insbesondere kann das Reaktionsprodukt ein oder mehrere 3-Hydroxybuttersäure-Polyolester (III), nämlich 3-Hydroxybuttersäure-Polyglycerinester, der allgemeinen Formel (IIIb)

R²O-CH₂-CH(OR²)-CH₂-[O-CH₂-CH(OR²)-CH₂]ₚ-OR² (IIIb)

umfassen,
wobei in der allgemeinen Formel (IIIb)
   - die Variable p eine ganze Zahl von 1 bis 4, insbesondere 1 oder 2, vorzugsweise 1, darstellt,
   - R², unabhängig voneinander, darstellt: Wasserstoff, einen Rest CH₃-CH(OH)-CH₂-C(O)- oder einen Rest CH₃-CH(OR³)-CH₂-C(O)- mit R³ = (C₁-C₂₉-Alkyl) - C(O) - oder einen Rest (C₁-C₂₉-Alkyl) - C(O) - , jedoch mit der Maßgabe, dass mindestens ein Rest R², insbesondere mindestens zwei Reste R², keinen Wasserstoff darstellt, und mit der Maßgabe, dass mindestens ein Rest R², insbesondere mindestens zwei Reste R², einen Rest CH₃-CH(OH)-CH₂-C(O)- oder einen Rest CH₃-CH(OR³)-CH₂-C(O)- mit R³ = (C₁-C₂₉-Alkyl) - C(O) - darstellt.

Insbesondere kann in diesem Zusammenhang in der allgemeinen Formel (IIIb) R², unabhängig voneinander, Wasserstoff oder einen Rest CH₃-CH(OH)-CH₂-C(O) - darstellen, jedoch mit der Maßgabe, dass mindestens ein Rest R², insbesondere mindestens zwei Reste R², keinen Wasserstoff darstellt.

Insbesondere kann in diesem Zusammenhang in der allgemeinen Formel (IIIb) R², unabhängig voneinander, darstellen: einen Rest CH₃-CH(OH)-CH₂-C(O)- oder einen Rest CH₃-CH(OR³)-CH₂-C(O)- mit R³ = (C₁-C₂₉-Alkyl) - C(O) - oder einen Rest (C₁-C₂₉-Alkyl) - C(O) - , jedoch mit der Maßgabe, dass mindestens ein Rest R², insbesondere mindestens zwei Reste R², einen Rest CH₃-CH(OH)-CH₂-C(O)- oder einen Rest CH₃-CH(OR³)-CH₂-C(O)- mit R³ = (C₁-C₂₉-Alkyl) - C(O) - darstellt.

Insbesondere kann in der allgemeinen Formel (IIIb) der C₁-C₂₉-Alkyl-Rest ein linearer (geradkettiger) oder verzweigter, gesättigter oder ein- oder mehrfach ungesättigter C₁-C₂₉-Alkylrest sein; bevorzugt kann der C₁-C₂₉-Alkyl-Rest ein C₁-C₉-Alkyl-Rest sein (insbesondere ein linearer oder verzweigter, gesättigter oder ein- oder mehrfach ungesättigter C₁-C₉-Alkylrest).

Gemäß einer besonderen Ausführungsform der vorliegenden Erfindung kann das Reaktionsprodukt insbesondere ein oder mehrere 3-Hydroxybuttersäure-Polyolester (III), nämlich 3-Hydroxybuttersäure-Polyglycerinester, der allgemeinen Formel (IIIc)

R²O-CH₂-CH(OR²)-CH₂-O-CH₂-CH(OR²)-CH₂-OR² (IIIc)

umfassen,
wobei in der allgemeinen Formel (IIIc) R², unabhängig voneinander, darstellt: Wasserstoff, einen Rest CH₃-CH(OH)-CH₂-C(O)- oder einen Rest CH₃-CH(OR³)-CH₂-C(O)- mit R³ = (C₁-C₂₉-Alkyl) - C(O) - oder einen Rest (C₁-C₂₉-Alkyl) - C(O) - , jedoch mit der Maßgabe, dass mindestens ein Rest R², insbesondere mindestens zwei Reste R², keinen Wasserstoff darstellt, und mit der Maßgabe, dass mindestens ein Rest R², insbesondere mindestens zwei Reste R², einen Rest CH₃-CH(OH)-CH₂-C(O)- oder einen Rest CH₃-CH(OR³)-CH₂-C(O) - mit R³ = (C₁-C₂₉-Alkyl) - C(O) - darstellt.

Insbesondere kann in der allgemeinen Formel (IIIc) R², unabhängig voneinander, Wasserstoff oder einen Rest CH₃-CH(OH)-CH₂-C(O)- darstellen, jedoch mit der Maßgabe, dass mindestens ein Rest R², insbesondere mindestens zwei Reste R², keinen Wasserstoff darstellt.

Insbesondere kann in der allgemeinen Formel (IIIc) R², unabhängig voneinander, darstellen: einen Rest CH₃-CH(OH)-CH₂-C(O)- oder einen Rest CH₃-CH(OR³)-CH₂-C(O)- mit R³ = (C₁-C₂₉-Alkyl) - C(O) - oder einen Rest (C₁-C₂₉-Alkyl) - C(O) - , jedoch mit der Maßgabe, dass mindestens ein Rest R², insbesondere mindestens zwei Reste R², einen Rest CH₃-CH(OH)-CH₂-C(O) - oder einen Rest CH₃-CH(OR³)-CH₂-C(O)- mit R³ = (C₁-C₂₉-Alkyl) - C(O) - darstellt.

Insbesondere kann in der allgemeinen Formel (IIIc) der C₁-C₂₉-Alkyl-Rest ein linearer (geradkettiger) oder verzweigter, gesättigter oder ein- oder mehrfach ungesättigter C₁-C₂₉-Alkylrest sein; bevorzugt kann der C₁-C₂₉-Alkyl-Rest ein C₁-C₉-Alkyl-Rest sein (insbesondere ein linearer oder verzweigter, gesättigter oder ein- oder mehrfach ungesättigter C₁-C₉-Alkylrest).

Gemäß einer besonderen Ausführungsform kann das Reaktionsprodukt insbesondere ein Gemisch von mindestens zwei voneinander verschiedenen 3-Hydroxybuttersäure-Polyolestern (III), nämlich 3-Hydroxybuttersäure-Polyglycerinestern, insbesondere wie zuvor definiert, umfassen.

Gemäß einer weiteren besonderen Ausführungsform kann das Reaktionsprodukt insbesondere ein Gemisch von mindestens drei voneinander verschiedenen 3-Hydroxybuttersäure-Polyolestern (III), nämlich 3-Hydroxybuttersäure-Polyglycerinestern, insbesondere wie zuvor definiert, umfassen.

Weiterer Gegenstand der vorliegenden Erfindung ist ebenfalls ein 3-Hydroxybuttersäure-Polyolester, nämlich 3-Hydroxybuttersäure-Polyglycerinester,
wobei der 3-Hydroxybuttersäure-Polyolester der allgemeinen Formel (IIIb)

   R²O-CH₂-CH(OR²)-CH₂-[O-CH₂-CH(OR²)-CH₂]ₚ-OR² (IIIb)
entspricht, wobei in der allgemeinen Formel (IIIb)
   - die Variable p eine ganze Zahl von 1 bis 4, insbesondere 1 oder 2, vorzugsweise 1, darstellt,
   - R², unabhängig voneinander, darstellt: Wasserstoff, einen Rest CH₃-CH(OH)-CH₂-C(O)- oder einen Rest CH₃-CH(OR³)-CH₂-C(O)- mit R³ = (C₁-C₂₉-Alkyl) - C(O) - oder einen Rest (C₁-C₂₉-Alkyl) - C(O) - , jedoch mit der Maßgabe, dass mindestens ein Rest R², insbesondere mindestens zwei Reste R², keinen Wasserstoff darstellt, und mit der Maßgabe, dass mindestens ein Rest R², insbesondere mindestens zwei Reste R², einen Rest CH₃-CH(OH)-CH₂-C(O)- oder einen Rest CH₃-CH(OR³)-CH₂-C(O)- mit R³ = (C₁-C₂₉-Alkyl) - C(O) - darstellt.

Insbesondere kann in der allgemeinen Formel (IIIb) R², unabhängig voneinander, Wasserstoff oder einen Rest CH₃-CH(OH)-CH₂-C(O)- darstellen, jedoch mit der Maßgabe, dass mindestens ein Rest R², insbesondere mindestens zwei Reste R², keinen Wasserstoff darstellt.

Weiterhin kann in der allgemeinen Formel (IIIb) R², unabhängig voneinander, insbesondere darstellen: einen Rest CH₃-CH(OH)-CH₂-C(O)- oder einen Rest CH₃-CH(OR³)-CH₂-C(O)- mit R³ = (C₁-C₂₉-Alkyl) - C(O) - oder einen Rest (C₁-C₂₉-Alkyl) - C(O) - , jedoch mit der Maßgabe, dass mindestens ein Rest R², insbesondere mindestens zwei Reste R², einen Rest CH₃-CH(OH)-CH₂-C(O) - oder einen Rest CH₃-CH(OR³)-CH₂-C(O)- mit R³ = (C₁-C₂₉-Alkyl) - C(O) - darstellt.

Insbesondere kann in der allgemeinen Formel (IIIb) der C₁-C₂₉-Alkyl-Rest ein linearer (geradkettiger) oder verzweigter, gesättigter oder ein- oder mehrfach ungesättigter C₁-C₂₉-Alkylrest sein; bevorzugt kann der C₁-C₂₉-Alkyl-Rest ein C₁-C₉-Alkyl-Rest sein (insbesondere ein linearer oder verzweigter, gesättigter oder ein- oder mehrfach ungesättigter C₁-C₉-Alkylrest).

Wiederum weiterer Gegenstand der vorliegenden Erfindung ist ebenfalls ein 3-Hydroxybuttersäure-Polyolester, nämlich 3-Hydroxybuttersäure-Polyglycerinester, insbesondere wie zuvor definiert,
wobei der 3-Hydroxybuttersäure-Polyolester der allgemeinen Formel (llIc),

   R²O-CH₂-CH(OR²)-CH₂-O-CH₂-CH(OR²)-CH₂-OR² (IIIc)
entspricht, wobei in der allgemeinen Formel (IIIc) R², unabhängig voneinander, darstellt: Wasserstoff, einen Rest CH₃-CH(OH)-CH₂-C(O)- oder einen Rest CH₃-CH(OR³)-CH₂-C(O)- mit R³ = (C₁-C₂₉-Alkyl) - C(O) - oder einen Rest (C₁-C₂₉-Alkyl) - C(O) - , jedoch mit der Maßgabe, dass mindestens ein Rest R², insbesondere mindestens zwei Reste R², keinen Wasserstoff darstellt, und mit der Maßgabe, dass mindestens ein Rest R², insbesondere mindestens zwei Reste R², einen Rest CH₃-CH(OH)-CH₂-C(O)- oder einen Rest CH₃-CH(OR³)-CH₂-C(O) - mit R³ = (C₁-C₂₉-Alkyl) - C(O) - darstellt.

Insbesondere kann in der allgemeinen Formel (IIIc) R², unabhängig voneinander, Wasserstoff oder einen Rest CH₃- CH(OH) - CH₂- C(O) - darstellen, jedoch mit der Maßgabe, dass mindestens ein Rest R², insbesondere mindestens zwei Reste R², keinen Wasserstoff darstellt.

Des Weiteren kann in der allgemeinen Formel (IIIc) R², unabhängig voneinander, insbesondere darstellen: einen Rest CH₃-CH(OH)-CH₂-C(O)- oder einen Rest CH₃-CH(OR³)-CH₂-C(O)- mit R³ = (C₁-C₂₉-Alkyl) - C(O) - oder einen Rest (C₁-C₂₉-Alkyl) - C(O) - , jedoch mit der Maßgabe, dass mindestens ein Rest R², insbesondere mindestens zwei Reste R², einen Rest CH₃-CH(OH)-CH₂-C(O) - oder einen Rest CH₃-CH(OR³)-CH₂-C(O)- mit R³ = (C₁-C₂₉-Alkyl) - C(O) - darstellt.

Insbesondere kann in der allgemeinen Formel (IIIc) der C₁-C₂₉-Alkyl-Rest ein linearer (geradkettiger) oder verzweigter, gesättigter oder ein- oder mehrfach ungesättigter C₁-C₂₉-Alkylrest sein; bevorzugt kann der C₁-C₂₉-Alkyl-Rest ein C₁-C₉-Alkyl-Rest sein (insbesondere ein linearer oder verzweigter, gesättigter oder ein- oder mehrfach ungesättigter C₁-C₉-Alkylrest).

Weiterer Gegenstand der vorliegenden Erfindung gemäß diesem Erfindungsaspekt ist entsprechend einer besonderen Ausführungsform ein Gemisch, welches mindestens zwei voneinander verschiedene 3-Hydroxybuttersäure-Polyolester, nämlich 3-Hydroxybuttersäure-Polyglycerinester, wie zuvor definiert, umfasst.

Wiederum weiterer Gegenstand der vorliegenden Erfindung gemäß diesem Erfindungsaspekt ist entsprechend einer weiteren besonderen Ausführungsform ein Gemisch, welches mindestens drei voneinander verschiedene 3-Hydroxybuttersäure-Polyolester, nämlich 3-Hydroxybuttersäure-Polyglycerinester, wie zuvor definiert, umfasst.

Das nach dem erfindungsgemäßen Verfahren erhältliche bzw. erfindungsgemäße Reaktionsprodukt, wie zuvor definiert, und/oder der nach dem erfindungsgemäßen Herstellungsverfahren erhältliche bzw. erfindungsgemäße 3-Hydroxybuttersäure-Polyolester, nämlich 3-Hydroxybuttersäure-Polyglycerinester, wie zuvor definiert, und/oder das nach dem erfindungsgemäßen Herstellungsverfahren erhältliche bzw. erfindungsgemäße Gemisch, wie zuvor definiert, weist gegenüber dem Stand der Technik eine Vielzahl von Vorteilen und Besonderheiten auf:
Wie die Anmelderin überraschend herausgefunden hat, eignet sich das nach dem erfindungsgemäßen Verfahren erhältliche bzw. erfindungsgemäße Reaktionsprodukt, wie zuvor definiert, und/oder der nach dem erfindungsgemäßen Herstellungsverfahren erhältliche bzw. erfindungsgemäße 3-Hydroxybuttersäure-Polyolester, nämlich 3-Hydroxybuttersäure-Polyglycerinester, wie zuvor definiert, und/oder das nach dem erfindungsgemäßen Herstellungsverfahren erhältliche bzw. erfindungsgemäße Gemisch, wie zuvor definiert, insbesondere als Präkursor bzw. Metabolit von 3-Hydroxybuttersäure bzw. deren Salzen, da dieser bzw. dieses einerseits physiologisch, insbesondere im Magen/Darm-Trakt, zu 3-Hydroxybuttersäure bzw. deren Salzen umgesetzt wird und andererseits gleichzeitig eine gute physiologische Kompatibilität bzw. Verträglichkeit aufweist, insbesondere im Hinblick auf Nichttoxizität und akzeptable organoleptische Eigenschaften.

Darüber hinaus ist das nach dem erfindungsgemäßen Verfahren erhältliche bzw. erfindungsgemäße Reaktionsprodukt, wie zuvor definiert, und/oder der nach dem erfindungsgemäßen Herstellungsverfahren erhältliche bzw. erfindungsgemäße 3-Hydroxybuttersäure-Polyolester, nämlich 3-Hydroxybuttersäure-Polyglycerinester, wie zuvor definiert, und/oder das nach dem erfindungsgemäßen Herstellungsverfahren erhältliche bzw. erfindungsgemäße Gemisch, wie zuvor definiert, ohne Weiteres auf synthetischem Wege auch in großtechnischem Maßstab zugänglich bzw. verfügbar, und zwar auch mit der erforderlichen pharmazeutischen bzw. pharmakologischen Qualität.

Zudem kann das nach dem erfindungsgemäßen Verfahren erhältliche bzw. erfindungsgemäße Reaktionsprodukt, wie zuvor definiert, und/oder der nach dem erfindungsgemäßen Herstellungsverfahren erhältliche bzw. erfindungsgemäße 3-Hydroxybuttersäure-Polyolester, nämlich 3-Hydroxybuttersäure-Polyglycerinester, wie zuvor definiert, und/oder das nach dem erfindungsgemäßen Herstellungsverfahren erhältliche bzw. erfindungsgemäße Gemisch, wie zuvor definiert, erforderlichenfalls in enantiomerenreiner bzw. enantiomerenangereicherter Form bereitgestellt werden.

Das nach dem erfindungsgemäßen Verfahren erhältliche bzw. erfindungsgemäße Reaktionsprodukt, wie zuvor definiert, und/oder der nach dem erfindungsgemäßen Herstellungsverfahren erhältliche bzw. erfindungsgemäße 3-Hydroxybuttersäure-Polyolester, nämlich 3-Hydroxybuttersäure-Polyglycerinester, wie zuvor definiert, und/oder das nach dem erfindungsgemäßen Herstellungsverfahren erhältliche bzw. erfindungsgemäße Gemisch, wie zuvor definiert, stellt somit ein effizientes pharmakologisches Wirkstoff-Target im Rahmen einer Ketokörper-Therapie des menschlichen oder tierischen Körpers dar.

Nachfolgend werden noch die übrigen Erfindungsaspekte weiterführende erläutert und im Detail beschrieben.

Weiterer Gegenstand der vorliegenden Erfindung - gemäß einem **dritten** Aspekt der vorliegenden Erfindung - ist eine pharmazeutische Zusammensetzung, insbesondere ein Arzneimittel oder Medikament, welche(s) ein nach dem erfindungsgemäßen Herstellungsverfahren erhältliches bzw. erfindungsgemäßes Reaktionsprodukt, wie zuvor definiert, und/oder einen nach dem erfindungsgemäßen Herstellungsverfahren erhältlichen bzw. erfindungsgemäßen 3-Hydroxybuttersäure-Polyolester, nämlich 3-Hydroxybuttersäure-Polyglycerinester, wie zuvor definiert, und/oder ein nach dem erfindungsgemäßen Herstellungsverfahren erhältliches bzw. erfindungsgemäßes Gemisch, wie zuvor definiert, umfasst.

Insbesondere betrifft die vorliegende Erfindung gemäß diesem Erfindungsaspekt eine pharmazeutische Zusammensetzung zur prophylaktischen und/oder therapeutischen Behandlung bzw. zur Verwendung bei der prophylaktischen und/oder therapeutischen Behandlung von Erkrankungen des menschlichen oder tierischen Körpers. Hierbei kann es sich insbesondere um Erkrankungen im Zusammenhang mit einer Störung des Energiestoffwechsels, insbesondere Ketokörperstoffwechsels, wie insbesondere Schädel-Hirn-Trauma, Schlaganfall, Hypoxien, kardiovaskuläre Erkrankungen wie Myokardinfarkt, Refeeding-Syndrom, Anorexien, Epilepsie, neurodegenerative Erkrankungen wie Demenz, Morbus Alzheimer, Morbus Parkinson, Multiple Sklerose und Amyotrophe Lateralsklerose, Fettstoffwechselerkrankungen wie Glucosetransporter-Defekt (GLUT1-Defekt), VL-FAOD und Mitochondriopathien wie mitochondrialer Thiolase-Defekt, Chorea Huntington, Krebserkrankungen wie T-Zell-Lymphome, Astrozytome und Glioblastome, HIV, rheumatische Erkrankungen wie rheumatoide Arthritis und Arthritis urica, Erkrankungen des Gastrointestinaltrakts wie chronisch entzündliche Darmerkrankungen, insbesondere Colitis ulcerosa und Morbus Crohn, lyosomale Speicherkrankheiten wie Sphingolipidosen, insbesondere Niemann-Pick-Erkrankung, Diabetes mellitus und Auswirkungen oder Nebenwirkungen von Chemotherapien, handeln.

Wiederum weiterer Gegenstand der vorliegenden Erfindung - gemäß einem **v i e r t e n** Aspekt der vorliegenden Erfindung - ist ein nach dem erfindungsgemäßen Herstellungsverfahren erhältliches bzw. erfindungsgemäßes Reaktionsprodukt, wie zuvor definiert, und/oder ein nach dem erfindungsgemäßen Herstellungsverfahren erhältlicher bzw. erfindungsgemäßer 3-Hydroxybuttersäure-Polyolester, nämlich 3-Hydroxybuttersäure-Polyglycerinester, wie zuvor definiert, und/oder ein nach dem erfindungsgemäßen Herstellungsverfahren erhältliches bzw. erfindungsgemäßes Gemisch, wie zuvor definiert, zur prophylaktischen und/oder therapeutischen Behandlung bzw. zur Verwendung bei der prophylaktischen und/oder therapeutischen Behandlung von Erkrankungen des menschlichen oder tierischen Körpers, insbesondere Erkrankungen im Zusammenhang mit einer Störung des Energiestoffwechsels, insbesondere Ketokörperstoffwechsels, wie insbesondere Schädel-Hirn-Trauma, Schlaganfall, Hypoxien, kardiovaskuläre Erkrankungen wie Myokardinfarkt, Refeeding-Syndrom, Anorexien, Epilepsie, neurodegenerative Erkrankungen wie Demenz, Morbus Alzheimer, Morbus Parkinson, Multiple Sklerose und Amyotrophe Lateralsklerose, Fettstoffwechselerkrankungen wie Glucosetransporter-Defekt (GLUT1-Defekt), VL-FAOD und Mitochondriopathien wie mitochondrialer Thiolase-Defekt, Chorea Huntington, Krebserkrankungen wie T-Zell-Lymphome, Astrozytome und Glioblastome, HIV, rheumatische Erkrankungen wie rheumatoide Arthritis und Arthritis urica, Erkrankungen des Gastrointestinaltrakts wie chronisch entzündliche Darmerkrankungen, insbesondere Colitis ulcerosa und Morbus Crohn, lyosomale Speicherkrankheiten wie Sphingolipidosen, insbesondere Niemann-Pick-Erkrankung, Diabetes mellitus und Auswirkungen oder Nebenwirkungen von Chemotherapien.

Gleichermaßen eignet sich das nach dem erfindungsgemäßen Herstellungsverfahren erhältliche bzw. erfindungsgemäße Reaktionsprodukt, wie zuvor definiert, und/oder der nach dem erfindungsgemäßen Herstellungsverfahren erhältliche bzw. erfindungsgemäße 3-Hydroxybuttersäure-Polyolester, nämlich 3-Hydroxybuttersäure-Polyglycerinester, wie zuvor definiert, und/oder das nach dem erfindungsgemäßen Herstellungsverfahren erhältliche bzw. erfindungsgemäße Gemisch, wie zuvor definiert, für die Verwendung eines nach dem erfindungsgemäßen Herstellungsverfahren erhältlichen bzw. erfindungsgemäßen Reaktionsprodukts, wie zuvor definiert, und/oder eines nach dem erfindungsgemäßen Herstellungsverfahren erhältlichen bzw. erfindungsgemäßen 3-Hydroxybuttersäure-Polyolesters, nämlich 3-Hydroxybuttersäure-Polyglycerinesters, wie zuvor definiert, und/oder eines nach dem erfindungsgemäßen Herstellungsverfahren erhältlichen bzw. erfindungsgemäßen Gemischs, wie zuvor definiert, zur prophylaktischen und/oder therapeutischen Behandlung bzw. zur Herstellung eines Arzneimittels zur prophylaktischen und/oder therapeutischen Behandlung von Erkrankungen des menschlichen oder tierischen Körpers, insbesondere Erkrankungen im Zusammenhang mit einer Störung des Energiestoffwechsels, insbesondere Ketokörperstoffwechsels, wie insbesondere Schädel-Hirn-Trauma, Schlaganfall, Hypoxien, kardiovaskuläre Erkrankungen wie Myokardinfarkt, Refeeding-Syndrom, Anorexien, Epilepsie, neurodegenerative Erkrankungen wie Demenz, Morbus Alzheimer, Morbus Parkinson, Multiple Sklerose und Amyotrophe Lateralsklerose, Fettstoffwechselerkrankungen wie Glucosetransporter-Defekt (GLUT1-Defekt), VL-FAOD und Mitochondriopathien wie mitochondrialer Thiolase-Defekt, Chorea Huntington, Krebserkrankungen wie T-Zell-Lymphome, Astrozytome und Glioblastome, HIV, rheumatische Erkrankungen wie rheumatoide Arthritis und Arthritis urica, Erkrankungen des Gastrointestinaltrakts wie chronisch entzündliche Darmerkrankungen, insbesondere Colitis ulcerosa und Morbus Crohn, lyosomale Speicherkrankheiten wie Sphingolipidosen, insbesondere Niemann-Pick-Erkrankung, Diabetes mellitus und Auswirkungen oder Nebenwirkungen von Chemotherapien.

Gleichermaßen eignet sich das nach dem erfindungsgemäßen Herstellungsverfahren erhältliche bzw. erfindungsgemäße Reaktionsprodukt, wie zuvor definiert, und/oder der nach dem erfindungsgemäßen Herstellungsverfahren erhältliche bzw. erfindungsgemäße 3-Hydroxybuttersäure-Polyolester, nämlich 3-Hydroxybuttersäure-Polyglycerinester, wie zuvor definiert, und/oder das nach dem erfindungsgemäßen Herstellungsverfahren erhältliche bzw. erfindungsgemäße Gemisch, wie zuvor definiert, für die Verwendung eines nach dem erfindungsgemäßen Herstellungsverfahren erhältlichen bzw. erfindungsgemäßen Reaktionsprodukts, wie zuvor definiert, und/oder eines nach dem erfindungsgemäßen Herstellungsverfahren erhältlichen bzw. erfindungsgemäßen 3-Hydroxybuttersäure-Polyolesters, nämlich 3-Hydroxybuttersäure-Polyglycerinesters, wie zuvor definiert, und/oder eines nach dem erfindungsgemäßen Herstellungsverfahren erhältlichen bzw. erfindungsgemäßen Gemischs, wie zuvor definiert, zur prophylaktischen und/oder therapeutischen Behandlung bzw. zur Herstellung eines Arzneimittels zur prophylaktischen und/oder therapeutischen Behandlung bzw. zur Anwendung von/bei katabolen Stoffwechsellagen, wie Hunger, Diäten oder kohlenhydratarmer Ernährung.

Gleichermaßen weiterer Gegenstand der vorliegenden Erfindung - gemäß einem **fünften** Aspekt der vorliegenden Erfindung - ist ein Nahrungsmittel- und/oder Lebensmittelerzeugnis, welches ein nach dem erfindungsgemäßen Herstellungsverfahren erhältliches bzw. erfindungsgemäßes Reaktionsprodukt, wie zuvor definiert, und/oder einen nach dem erfindungsgemäßen Herstellungsverfahren erhältlichen bzw. erfindungsgemäßen 3-Hydroxybuttersäure-Polyolester, nämlich 3-Hydroxybuttersäure-Polyglycerinester, wie zuvor definiert, und/oder ein nach dem erfindungsgemäßen Herstellungsverfahren erhältliches bzw. erfindungsgemäßes Gemisch, wie zuvor definiert, umfasst.

Gemäß einer besonderen Ausführungsform kann das Nahrungsmittel- und/oder Lebensmittelerzeugnis insbesondere ein Nahrungsergänzungsmittel, ein funktionelles Lebensmittel (*Functional Food*), ein *Novel Food,* ein Lebensmittelzusatzstoff, ein Nahrungszusatz, ein diätetisches Lebensmittel, ein Power-Snack, ein Appetitzügler oder ein Kraft- und/oder Ausdauersport-Supplement sein.

Schließlich ist wiederum weiterer Gegenstand der vorliegenden Erfindung - gemäß einem **sechsten** Aspekt der vorliegenden Erfindung - die nicht therapeutische Verwendung eines nach dem erfindungsgemäßen Herstellungsverfahren erhältlichen Reaktionsprodukts, wie zuvor definiert, und/oder eines nach dem erfindungsgemäßen Herstellungsverfahren erhältlichen Gemischs, wie zuvor definiert, in einem Nahrungsmittel- und/oder Lebensmittelerzeugnis.

Gemäß diesem Erfindungsaspekt kann das Nahrungsmittel- und/oder Lebensmittelerzeugnis insbesondere ein Nahrungsergänzungsmittel, ein funktionelles Lebensmittel (*Functional Food*), ein *Novel Food,* ein Lebensmittelzusatzstoff, ein Nahrungszusatz, ein diätetisches Lebensmittel, ein Power-Snack, ein Appetitzügler oder ein Kraft- und/oder Ausdauersport-Supplement sein.

Die vorliegende Erfindung wird anhand der nachfolgenden Ausführungsbeispiele veranschaulicht, welche die vorliegende Erfindung jedoch keinesfalls beschränken sollen, sondern lediglich die beispielhafte und nichtlimitierende Durchführungsweise und Ausgestaltung der vorliegenden Erfindung erläutern sollen.

### AUSFÜHRUNGSBEISPIELE:

### Verwendete Abkürzungen

- 3-BHB-Ethyl = 3-Hydroxybuttersäureethylester (Edukt)
- 3-BHB-FS = 3-Hydroxybuttersäure (Reaktionsnebenprodukt)
- PG(2) = Diglycerin: HO-CH₂-CH(OH)-CH₂-O-CH₂-CH(OH)-CH₂-OH
- n.d. = nicht bestimmt
- 3-BHB-Dimer = Reaktionsnebenprodukt der nachstehenden Formel:

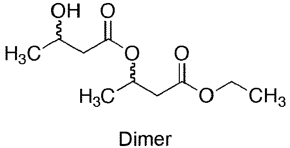

### Herstellungsbeispiele

Das erfindungsgemäße Herstellungsverfahren wird anhand der nachfolgenden Ausführungsbeispiele veranschaulicht. Das diesbezügliche allgemeine Reaktionsschema hierzu ist im allgemeinen Beschreibungsteil dargestellt und erläutert.

### Herstellung von 3-BHB-Diglycerinester-Gemischen

In einem 4.000-ml-Mehrhalskolben mit Dephlegmator (Partialkondensator) und Destillationsbrücke werden 3.000 g (R)/(S)-3-Hydroxybuttersäureethylester (racemisch), 450 g Diglycerin und 14 g immobilisiertem Enzym (CALB-Lipase auf Polymerträger, abgeleitet von *Candida antarctica,* z. B. Novozym^{®} 435 von der Fa. Sigma-Aldrich bzw. Merck oder Lipozym^{®} 435 von der Fa. Strem Chemicals, Inc.) vorgelegt.

Das Reaktionsgemisch wird bei 70 °C und unter Vakuum (< 500 mbar) für 50 h gerührt. Danach wird das Enzym abfiltriert und der überschüssige 3-Hydroxybuttersäureethylester unter Vakuum abdestilliert. Der erhaltene Rückstand wird für 2 bis 4 Stunden im Hochvakuum dampfbehandelt (Dampftemperatur: 160 °C).

Der Rückstand besteht nach analytischer Untersuchung aus 17 % 3-BHB-Mono-Diglycerinester, 46 % 3-BHB-Di-Diglycerinester, 22 % 3-BHB-Tri-Diglycerinester, 2 % 3-BHB-Tetra-Diglycerinester, 2 % 3-Hydroxybuttersäure, 7 % 3-Hydroxybuttersäure-Dimer und 2 % Diglycerin. Die Charakterisierung erfolgt mittels GC, GPC und GC-MS.

Im Rahmen der Aufreinigung werden Edukte und Reaktionsnebenprodukte entfernt, so dass ein Reingemisch erhalten wird. Ein Teil des Gemischs wird einer Auftrennung mittels Chromatographie unterzogen, so dass die verschiedenen Diglycerinester jeweils als Reinstoffe erhalten werden (d. h. jeweils reiner 3-BHB-Mono-Diglycerinester, reiner 3-BHB-Di-Diglycerinester, reiner 3-BHB-Tri-Diglycerinester und reiner 3-BHB-Tetra-Diglycerinester). Ein anderer Teil des Gemischs wird einer Auftrennung mittels fraktionierter Destillation unterzogen.

### Weitere von Herstellung von 3-BHB-Diglycerinester-Gemischen

Der vorangehende Versuch wird wiederholt, jedoch mit Natriummethylat (NaOMe) als Katalysator (1 Gew.-%) anstelle des Enzyms und bei Temperaturen zwischen 100 und 120 °C (40 Mol-% Überschuss 3-BHB-Ethylester). Es werden vergleichbare Ergebnisse erhalten. Aufreinigung und Trennung bzw. Fraktionierung erfolgen in gleicher Weise.

### Weitere von Herstellung von 3-BHB-Diglycerinester-Gemischen

Der vorangehende Versuch mit mit Natriummethylat (NaOMe) als Katalysator wird wiederholt, jedoch mit anderen Polyolen (nämlich mit Polyglycerin PG(3) und mit 1,2-Pentandiol). Es werden vergleichbare Ergebnisse erhalten. Aufreinigung und Trennung bzw. Fraktionierung erfolgen in gleicher Weise.

### Weitere Herstellungsbeispiele

Es werden verschiedene Polyol-Komponenten auf Basis mehrwertiger Alkohole mit 3-BHB-Ethylester enzymatisch umgesetzt.

Als Polyalkohole werden 1,2-Pentandiol (nicht erfindungsgemäß) und Diglycerin PG(2) ausgewählt. Die jeweiligen Alkohole werden bei 70 °C für 24 h mit immobilisiertem Enzym (CALB-Lipase auf Polymerträger, abgeleitet von *Candida antarctica,* z. B. Novozym^{®} 435 von der Fa. Sigma-Aldrich bzw. Merck oder Lipozym^{®} 435 von der Fa. Strem Chemicals, Inc.) umgesetzt (jeweils 1 Gew.-% Enzym und jeweils 40 Mol-% Überschuss 3-BHB-Ethylester).

Die vorgenannten Polyalkohole 1,2-Pentandiol und Diglycerin PG(2) werden mit den vorgenannten Enzymen effizient zu den gewünschten Produkten umgesetzt. Es werden vergleichbare Ergebnisse zu den vorangehenden Versuchen erhalten. Aufreinigung und Trennung bzw. Fraktionierung erfolgen in gleicher Weise.

Die Versuche werden mit Natriummethylat (NaOMe) als Katalysator anstelle der Enzyme und bei Temperaturen zwischen 100 und 120 °C wiederholt. Es werden vergleichbare Ergebnisse erhalten. Aufreinigung und Trennung bzw. Fraktionierung erfolgen in gleicher Weise.

Da insbesondere die 3-BHB-PG(2)-Ester ein nur wenig bitteren Geschmack aufweisen, sind vor allem diese Ester eine effiziente Produktgruppe für eine therapeutische Anwendung. Daher wird der vorangehende Versuch mit Enzym und Diglycerin PG(2) als Polyalkohol in einem größeren Maßstab (2 bis 4 kg) durchgeführt.

Zunächst werden in einem Maßstab von 2 kg die stöchiometrischen Reaktionsbedingungen der vorangehenden Versuche angewendet (40 Mol-% Überschuss 3-BHB-Ethylester, 1 Gew.-% Enzym). Nach 15 h wird ein Teil der Reaktionsmischung (ca. 200 g) für eine weitere Untersuchung entnommen. Hierbei handelt es sich um ein Mono/Di-PG(2)-Ester-Gemisch. Danach werden weitere ca. 2 kg 3-BHB-Ethylester hinzugegeben. Die Menge entspricht einem Überschuss von 100 Mol-%, bereits berechnet auf das (R)-Enantiomer. Das Ziel ist die Herstellung eines Voll-Esters. Es ist zu erkennen, dass sich nach etwa 20 bis 30 h ein konstanter Gehalt an Di-PG(2)-Ester einstellt; der Mono-PG(2)-Ester Anteil fällt ab und der Tri-PG(2)-Ester-Anteil steigt. Weitere Analysen (GPC) zeigen, dass sich auch ein Tetra-PG(2)-Ester gebildet hat.

Nach Abdestillieren von überschüssigem 3-BHB-Ethylester weist das zunächst erhaltene (niedrigsiedende) Mono/Di-PG(2)-Ester-Gemisch nur einen leicht bitteren Geschmack auf, während das höhere (höhersiedende) Di-/Tri-/Tetra-PG(2)-Ester-Gemisch einen etwas stärker bitteren Geschmack aufweist. Beide Gemische sind jedoch organoleptisch akzeptabel und kompatibel. Des Weiteren handelt es sich noch um Rohester, welche noch z. B. 3-BHB-Dimer enthalten, welches als Reinstoff auch einen bitteren Geschmack aufweist.

Nach weitergehender Aufreinigung unter Entfernung restlicher Edukte und Reaktionsnebenprodukte wird ein Reingemisch mit signifikant verbesserten organoleptischen Eigenschaften erhalten.

### Wiederum weitere Herstellungsbeispiele

### Herstellung von 3-BHB-Diglycerinester-Gemischen aus Diglycerin und 3-Hydroxybuttersäureethylester mittels basischer Katalyse

In einem 1.000-ml-Mehrhalskolben mit Dephlegmator (Partialkondensator) und Destillationsbrücke werden 350 g (R)/(S)-3-Hydroxybuttersäureethylester (racemisch) und 110 g Diglycerin bei 80 °C unter N₂-Atmosphäre vorgelegt. Zur Reaktionsmischung werden 15 g einer 30 Gew.-%-igen Natriummethanolatlösung (NaOMe-Lösung) in Methanol bei 100 °C zu getropft. Danach wird das Vakuum schrittweise bis auf < 100 mbar verringert und die Reaktionsmischung für weitere 12 h gerührt. Anschließend wird der überschüssige 3-BHB-Ethylester bei verringertem Vakuum (< 20 mbar) abdestilliert. Das Reaktionsprodukt ist ein 3-BHB-Diglycerinester-Gemisch.

### Herstellung von 3-BHB-Diglycerinester-Gemischen aus Diglycerin und 3-Hydroxybuttersäureethylester mittels enzymatischer Katalyse

In einem 4.000-ml-Mehrhalskolben mit Dephlegmator (Partialkondensator) und Destillationsbrücke werden 3.000 g (R)/(S)-3-Hydroxybuttersäureethylester (racemisch), 450 g Diglycerin und 14 g immobilisiertes Enzym (CALB) vorgelegt. Das Reaktionsgemisch wird bei 70 °C und unter Vakuum (< 500 mbar) für 50 h gerührt. Danach wird das Enzym abfiltriert und der überschüssige 3-Hydroxybuttersäureethylester unter Vakuum abdestilliert. Der erhaltene Rückstand wird für 2 bis 4 h im Hochvakuum gedämpft (Dampftemperatur: 160°C). Der Rückstand besteht nach analytischer Untersuchung aus 17% Monodiglycerinester, 46 % Didiglycerinester, 22 % Tridiglycerinester, 2 % Tetradiglycerinester, 2 % 3-Hydroxybuttersäure, 7 % 3-Hydroxybuttersäuredimer und 2 % Diglycerin. Die Charakterisierung erfolgt mittels GC, GPC und GC-MS.

### Funktionalisierungsversuche

Das im vorangehenden Versuch erhaltene höhere (höhersiedende) Di-/Tri-PG(2)-Ester-Gemisch wird nachfolgend durch Umsetzung mit C₇-Anhydrid funktionalisiert, um vollständig an allen OH-Gruppen veresterte Produkte zu erhalten. Das diesbezügliche allgemeine Reaktionsschema hierzu ist im allgemeinen Beschreibungsteil dargestellt und erläutert.

Die Versuche zeigen, dass die beabsichtigte Funktionalisierung durch Umsetzung mit C₇-Anhydrid zu den gewünschten Produkten führt (d. h. Veresterung der freien OH-Gruppen), wie durch entsprechende Analytik bestätigt.

Vergleichbare Funktionalisierungsversuche werden auch mit höheren Carbonsäureanhydriden (jeweils mit C₁₀-, C₂₀- und C₂₈-Carbonsäureanhydriden) durchgeführt und führen zu analogen Ergebnissen (d. h. Veresterung der freien OH-Gruppen), wie durch entsprechende Analytik bestätigt.

### Physiologische Anwendunasversuche: in-vitro-Verdauversuche

### Verdauversuche (Spalt-bzw. Spaltungsversuche) von erfindunasaemäßen 3-BHB-PG(2)-Ester-Gemischen

Mittels Spaltungsversuchen wird gezeigt, dass erfindungsgemäß hergestellte 3-BHB-PG(2)-Ester bzw. deren Gemische, einschließlich der Reaktionsnebenprodukte wie Dimeren etc., im menschlichen gastrointestinalen Trakt gespalten werden können.

Als Ausgangsgemisch wird einerseits ein aufgereinigtes, nach dem erfindungsgemäßen Verfahren erhaltenes Gemisch aus 3-BHB-Monodiglycerinester, 3-BHB-Di-Diglycerinester, 3-BHB-Tri-Diglycerinester und 3-BHB-Tetra-Diglycerinester und andererseits ein aufgereinigtes, nach dem erfindungsgemäßen Verfahren erhaltenes Gemisch aus 3-BHB-Mono-Diglycerinester und 3-BHB-Di-Diglycerinester eingesetzt.

Für die Spaltungsversuche unter köpernahen Bedingungen werden zwei Medien untersucht:
- FaSSGF, welches den Magen simuliert
- FaSSIF, welches den Darmtrakt simuliert

Beide Medien stammen von der Firma Biorelevant^{®}, Ltd., Großbritannien. Zusätzlich wird in einigen Experimenten beiden Medien jeweils Schweine-Pankrease zugesetzt (Panzytrat^{®} 40.000, Fa. Allergan).

Die Ergebnisse der Spaltungsversuche in einem FaSSGF- bzw. FaSSIF-Medium mit Panzytrat^{®} und ohne Panzytrat^{®} (jeweils 35 °C, 24 h) zeigen, dass die Proben unter FaSSGF-Bedingungen mit Panzytrat^{®} und ohne Panzytrat^{®} hydrolysieren; dies liegt hauptsächlich am niedrigen pH-Wert (pH = 1,6) des Mediums. Bei FaSSIF-Bedingungen findet eine geringere Umsetzung unter Verwendung von Panzytrat^{®} statt.

Bei allen Experimenten ist zu erkennen, dass sich die Kaskade (Tetraester wird zu Triester, Triester wird zu Diester etc.) bis zu der gewünschten freien Säure 3-BHB bzw. 3-BHB-FS fortsetzt.

### Weitere Verdauversuche (Spaltungsversuche) von erfindunasaemäßen 3-BHB-PG(2)-Ester-Gemischen

### Spaltungsversuche mit Pankreatin

2 g eines wie zuvor beschrieben hergestellten Gemischs auf Basis von 3-BHB-Mono-Diglycerinester, 3-BHB-Di-Diglycerinester, 3-BHB-Tri-Diglycerinester und 3-BHB-Tetra-Diglycerinester werden in 50 g Wasser gelöst und mit 0,5 g (1 Gew.-%) Pankreatin versetzt. Das Pankreatin wird in Form des kommerziell verfügbaren Produkts Panzytrat^{®} 40.000 von der Fa. Allergan eingesetzt. Das Ganze wird auf einer Heizplatte bei 50 °C gerührt; der Reaktionsverlauf wird mittels kontinuierlicher Erfassung der Säurezahl über die Zeit ermittelt und verfolgt. Die Säurezahl steigt über den Beobachtungszeitraum an (Spaltung des 3-BHB-Diglycerinester-Gemischs zu der freien Säure). Der Umsatz/Zeit-Verlauf der wässrigen Spaltung des erfindungsgemäßen Estergemischs mittels Pankreatin, einschließlich Zunahme der Säurezahl über die Zeit, belegt die gewünschte Zersetzung des Eduktgemischs zu der freien Säure. Dies wird durch entsprechende Analytik bestätigt. Der Versuch belegt, dass das erfindungsgemäße Ausgangsgemisch ein geeigneter physiologischer Präkursor für 3-Hydroxybuttersäure für die entsprechenden Ketokörpertherapien darstellt.

Der Versuch wird jeweils anhand der einzelnen Ester in Reinform wiederholt und verifiziert. Es werden vergleichbare Ergebnisse erhalten, d. h. sowohl der 3-BHB-Mono-Diglycerinester als auch der 3-BHB-Di-Diglycerinester sowie auch der 3-BHB-Tri-Diglycerinester und der 3-BHB-Tetra-Diglycerinester werden durch Pankreatin jeweils zu der freien 3-Hydroxybuttersäure gespalten.

Die zuvor geschilderten Spaltungsversuche belegen, dass die Polyolester, insbesondere Polyglycerinester, der 3-Hydroxybuttersäure effiziente Präkursoren bzw. Metabolite der freien Hydroxybuttersäure bzw. deren Salzen darstellen, insbesondere im Hinblick auf ihre beabsichtigte Wirkung, welche in physiologisch verträglicher bzw. physiologisch kompatibler Form vorliegen.

## Patentansprüche

1. Verfahren zur Herstellung von Polyolestern, nämlich Polyglycerinestern, der 3-Hydroxybuttersäure (beta-Hydroxybuttersäure, BHB bzw. 3-BHB),
wobei mindestens eine Verbindung der allgemeinen Formel (I)
CH₃-CH(OH)-CH₂-C(O)OR¹ (I)
wobei in der allgemeinen Formel (I) der Rest R¹ Wasserstoff oder ein C₁-C₄-Alkyl, insbesondere ein C₁-C₄-Alkyl, bevorzugt Methyl oder Ethyl, besonders bevorzugt Ethyl, darstellt,
mit mindestens einem Polyglycerin der allgemeinen Formel (IIb)
HO-CH₂-CH(OH)-CH₂-[O-CH₂-CH(OH)-CH₂]ₚ-OH (IIb)
wobei in der allgemeinen Formel (IIb) die Variable p eine ganze Zahl von 1 bis 4, insbesondere 1 oder 2, vorzugsweise 1, darstellt,
umgesetzt wird,
wobei die Umsetzung in Abwesenheit von Lösemitteln und/oder ohne jedwedes Lösemittel durchgeführt wird und
wobei die Umsetzung in Gegenwart eines Katalysators durchgeführt wird,
so dass als Reaktionsprodukt ein oder mehrere 3-Hydroxybuttersäure-Polyolester (III), nämlich 3-Hydroxybuttersäure-Polyglycerinester, erhalten werden,
wobei bei der Umsetzung gleichzeitig die Verbindung gemäß der allgemeinen Formel (IV)
R¹-OH (IV)
gebildet wird, wobei in der allgemeinen Formel (IV) der Rest R¹ Wasserstoff oder ein C₁-C₄-Alkyl, insbesondere ein C₁-C₄-Alkyl, bevorzugt Methyl oder Ethyl, besonders bevorzugt Ethyl, darstellt, wobei die Verbindung gemäß der allgemeinen Formel (IV) der Umsetzung kontinuierlich entzogen wird.

2. Verfahren nach Anspruch 1,
wobei Verbindung der allgemeinen Formel (I) in racemischer Form oder in Form des (R)-Enantiomers eingesetzt wird; und/oder
wobei in der allgemeinen Formel (I) der Rest R¹ Ethyl darstellt und/oder
wobei als Verbindung der allgemeinen Formel (I) 3-Hydroxybuttersäureethylester (Ethyl-3-hydroxybutyrat) der Formel CH₃-CH(OH)-CH₂-C(O)OC₂H₅ eingesetzt wird; und/oder
wobei die Umsetzung in Gegenwart eines Enzyms und/oder eines metallhaltigen und/oder metallbasierten, sauren oder basischen Katalysators, vorzugsweise in Gegenwart eines Enzyms, durchgeführt wird; insbesondere wobei der Katalysators nach Umsetzung rezykliert wird; und/oder
wobei die Verbindung der allgemeinen Formel (I), bezogen auf die Hydroxylgruppen des Polyglycerins (IIb) in molaren Mengen in einem Bereich von äquimolarer Menge bis zu einem molaren Überschuss von 200 Mol-%, insbesondere in einem Bereich von äquimolarer Menge bis zu einem molaren Überschuss von 150 Mol-%, vorzugsweise in einem Bereich von äquimolarer Menge bis zu einem molaren Überschuss von 100 Mol%, eingesetzt wird; und/oder
wobei die Verbindung der allgemeinen Formel (I) und das Polyglycerin (IIb) in einem Molverhältnis von Verbindung der allgemeinen Formel (I) / Polyglycerin (IIb) in einem Bereich von 1:1 bis 10 : 1, insbesondere in einem Bereich von 2 : 1 bis 8 : 1, vorzugsweise in einem Bereich von 3 : 1 bis 6 : 1, eingesetzt werden; und/oder
wobei das Polyglycerin (IIb) ein Diglycerin der Formel (IIc)
HO-CH₂-CH(OH)-CH₂-O-CH₂-CH(OH)-CH₂-OH (IIc)
ist.

3. Verfahren nach einem Anspruch 1 oder Anspruch 2,
wobei das erhaltene Reaktionsprodukt nach erfolgter Umsetzung fraktioniert wird, insbesondere destillativ fraktioniert wird; und/oder
wobei nichtumgesetzte Ausgangsverbindungen (I) und/oder (IIb) aus dem Reaktionsprodukt abgetrennt und anschließend rezykliert werden; und/oder
wobei im Reaktionsprodukt nach erfolgter Umsetzung noch vorhandene Hydroxylgruppen zumindest teilweise, vorzugsweise vollständig, funktionalisiert, insbesondere verestert werden; und/oder wobei sich der Umsetzung eine teilweise, insbesondere vollständige Funktionalisierung, insbesondere Veresterung, noch vorhandener Hydroxylgruppen anschließt; insbesondere wobei die Funktionalisierung, insbesondere Veresterung, der Hydroxylgruppen durch Reaktion mit einem Carbonsäureanhydrid, insbesondere C₂-C₃₀-Carbonsäureanhydrid, bevorzugt C₂-C₁₀-Carbonsäureanhydrid, vorzugsweise C₇-Carbonsäureanhydrid, durchgeführt wird, insbesondere wobei das C₂-C₃₀-Carbonsäureanhydrid, bevorzugt C₂-C₁₀-Carbonsäureanhydrid, vorzugsweise C₇-Carbonsäureanhydrid, ein lineares (geradkettiges) oder verzweigtes, gesättigtes oder ein- oder mehrfach ungesättigtes C₂-C₃₀-Carbonsäureanhydrid, bevorzugt C₂-C₁₀-Carbonsäureanhydrid, vorzugsweise C₇-Carbonsäureanhydrid, ist.

4. Reaktionsprodukt, nämlich 3-Hydroxybuttersäure-Polyglycerinester, erhältlich gemäß dem Verfahren nach einem der Ansprüche 1 bis 3.

5. 3-Hydroxybuttersäure-Polyolester, nämlich 3-Hydroxybuttersäure-Polyglycerinester,
wobei der 3-Hydroxybuttersäure-Polyolester der allgemeinen Formel (IIIb)
R²O-CH₂-CH(OR²)-CH₂-[O-CH₂-CH(OR²)-CH₂]ₚ-OR² (IIIb)
entspricht, wobei in der allgemeinen Formel (IIIb)
• die Variable p eine ganze Zahl von 1 bis 4, insbesondere 1 oder 2, vorzugsweise 1, darstellt,
• R², unabhängig voneinander, darstellt: Wasserstoff, einen Rest CH₃-CH(OH)-CH₂-C(O)- oder einen Rest CH₃-CH(OR³)-CH₂-C(O)- mit R³ = (C₁-C₂₉-Alkyl) - C(O) - oder einen Rest (C₁-C₂₉-Alkyl) - C(O) - , jedoch mit der Maßgabe, dass mindestens ein Rest R², insbesondere mindestens zwei Reste R², keinen Wasserstoff darstellt, und mit der Maßgabe, dass mindestens ein Rest R², insbesondere mindestens zwei Reste R², einen Rest CH₃- CH(OH) - CH₂-C(O) - oder einen Rest CH₃-CH(OR³)-CH₂-C(O)- mit R³ = (C₁-C₂₉-Alkyl) - C(O) - darstellt.

6. 3-Hydroxybuttersäure-Polyolester nach Anspruch 5,
wobei in der allgemeinen Formel (IIIb) R², unabhängig voneinander, Wasserstoff oder einen Rest CH₃-CH(OH)-CH₂-C(O)- darstellt, jedoch mit der Maßgabe, dass mindestens ein Rest R², insbesondere mindestens zwei Reste R², keinen Wasserstoff darstellt.

7. 3-Hydroxybuttersäure-Polyolester nach Anspruch 5,
wobei in der allgemeinen Formel (IIIb) R², unabhängig voneinander, darstellt: einen Rest CH₃-CH(OH)-CH₂-C(O)- oder einen Rest CH₃-CH(OR³)-CH₂-C(O)- mit R³ = (C₁-C₂₉-Alkyl) - C(O) - oder einen Rest (C₁-C₂₉-Alkyl) - C(O) - , jedoch mit der Maßgabe, dass mindestens ein Rest R², insbesondere mindestens zwei Reste R², einen Rest CH₃-CH(OH)-CH₂-C(O)- oder einen Rest CH₃-CH(OR³)-CH₂-C(O)- mit R³ = (C₁-C₂₉-Alkyl) - C(O) - darstellt.

8. 3-Hydroxybuttersäure-Polyolester nach einem der Ansprüche 5 bis 7,
wobei der 3-Hydroxybuttersäure-Polyolester der allgemeinen Formel (llIc),
R²O-CH₂-CH(OR²)-CH₂-O-CH₂-CH(OR²)-CH₂-OR² (IIIc)
entspricht, wobei in der allgemeinen Formel (IIIc) R², unabhängig voneinander, darstellt: Wasserstoff, einen Rest CH₃-CH(OH)-CH₂-C(O)- oder einen Rest CH₃-CH(OR³)-CH₂-C(O)- mit R³ = (C₁-C₂₉-Alkyl) - C(O) - oder einen Rest (C₁-C₂₉-Alkyl) - C(O) - , jedoch mit der Maßgabe, dass mindestens ein Rest R², insbesondere mindestens zwei Reste R², keinen Wasserstoff darstellt, und mit der Maßgabe, dass mindestens ein Rest R², insbesondere mindestens zwei Reste R², einen Rest CH₃-CH(OH)-CH₂-C(O)- oder einen Rest CH₃-CH(OR³)-CH₂-C(O)- mit R³ = (C₁-C₂₉-Alkyl)-C(O)- darstellt.

9. 3-Hydroxybuttersäure-Polyolester nach Anspruch 8,
wobei in der allgemeinen Formel (IIIc) R², unabhängig voneinander, Wasserstoff oder einen Rest CH₃-CH(OH)-CH₂-C(O)- darstellt, jedoch mit der Maßgabe, dass mindestens ein Rest R², insbesondere mindestens zwei Reste R², keinen Wasserstoff darstellt

10. 3-Hydroxybuttersäure-Polyolester nach Anspruch 8,
wobei in der allgemeinen Formel (IIIc) R², unabhängig voneinander, darstellt: einen Rest CH₃-CH(OH)-CH₂-C(O)- oder einen Rest CH₃-CH(OR³)-CH₂-C(O)- mit R³ = (C₁-C₂₉-Alkyl) - C(O) - oder einen Rest (C₁-C₂₉-Alkyl) - C(O) - , jedoch mit der Maßgabe, dass mindestens ein Rest R², insbesondere mindestens zwei Reste R², einen Rest CH₃-CH(OH)-CH₂-C(O)- oder einen Rest CH₃-CH(OR³)-CH₂-C(O)- mit R³ = (C₁-C₂₉-Alkyl) - C(O) - darstellt.

11. Gemisch, umfassend mindestens zwei oder drei voneinander verschiedene 3-Hydroxybuttersäure-Polyolester, nämlich 3-Hydroxybuttersäure-Polyglycerinester, wie in einem der Ansprüche 5 bis 10 definiert.

12. Pharmazeutische Zusammensetzung, insbesondere Arzneimittel oder Medikament, umfassend ein Reaktionsprodukt gemäß Anspruch 4 und/oder einen 3-Hydroxybuttersäure-Polyolester gemäß einem der Ansprüche 5 bis 10 und/oder ein Gemisch gemäß Anspruch 11.

13. Reaktionsprodukt gemäß Anspruch 4 und/oder 3-Hydroxybuttersäure-Polyolester gemäß einem der Ansprüche 5 bis 10 und/oder Gemisch gemäß Anspruch 11 zur Verwendung bei der prophylaktischen und/oder therapeutischen Behandlung von Erkrankungen des menschlichen oder tierischen Körpers, insbesondere Erkrankungen im Zusammenhang mit einer Störung des Energiestoffwechsels, insbesondere Ketokörperstoffwechsels, wie insbesondere Schädel-Hirn-Trauma, Schlaganfall, Hypoxien, kardiovaskuläre Erkrankungen wie Myokardinfarkt, Refeeding-Syndrom, Anorexien, Epilepsie, neurodegenerative Erkrankungen wie Demenz, Morbus Alzheimer, Morbus Parkinson, Multiple Sklerose und Amyotrophe Lateralsklerose, Fettstoffwechselerkrankungen wie Glucosetransporter-Defekt (GLUT1-Defekt), VL-FAOD und Mitochondriopathien wie mitochondrialer Thiolase-Defekt, Chorea Huntington, Krebserkrankungen wie T-Zell-Lymphome, Astrozytome und Glioblastome, HIV, rheumatische Erkrankungen wie rheumatoide Arthritis und Arthritis urica, Erkrankungen des Gastrointestinaltrakts wie chronisch entzündliche Darmerkrankungen, insbesondere Colitis ulcerosa und Morbus Crohn, lyosomale Speicherkrankheiten wie Sphingolipidosen, insbesondere Niemann-Pick-Erkrankung, Diabetes mellitus und Auswirkungen oder Nebenwirkungen von Chemotherapien.

14. Nahrungsmittel- und/oder Lebensmittelerzeugnis, umfassend ein Reaktionsprodukt gemäß Anspruch 4 und/oder einen 3-Hydroxybuttersäure-Polyolester gemäß einem der Ansprüche 5 bis 10 und/oder ein Gemisch gemäß Anspruch 11.

15. Nicht therapeutische Verwendung eines Reaktionsprodukts gemäß Anspruch 4 und/oder eines 3-Hydroxybuttersäure-Polyolesters gemäß einem der Ansprüche 5 bis 10 und/oder eines Gemischs gemäß Anspruch 11 in einem Nahrungsmittel- und/oder Lebensmittelerzeugnis.

## Claims

1. A process for the preparation of polyol esters, namely polyglycerol esters of 3-hydroxybutyric acid (beta-hydroxybutyric acid, BHB or 3-BHB),
wherein at least one compound of the general formula (I)
CH₃-CH(OH)-CH₂-C(O)OR¹ (I)
where, in the general formula (I), the radical R¹ is hydrogen or a C₁-C₄-alkyl, in particular a C₁-C₄-alkyl, preferably methyl or ethyl, particularly preferably ethyl,
is reacted with at least one polyglycerol of the general formula (IIb)
HO-CH₂-CH(OH)-CH₂-[O-CH₂-CH(OH)-CH₂]ₚ-OH (IIb)
in which general formula (IIb) the variable p represents an integer from 1 to 4, in particular 1 or 2, preferably 1,
wherein the reaction is carried out in the absence of solvents and/or without any solvent, and
wherein the reaction is carried out in the presence of a catalyst,
such that one or more 3-hydroxybutyric acid polyol esters (III), namely 3-hydroxybutyric acid polyglycerol esters, are obtained as reaction product,
wherein the reaction simultaneously yields the compound according to the general formula (IV)
R¹-OH (IV)
in which general formula (IV) the radical R¹ represents hydrogen or a C₁-C₄-alkyl, in particular a C₁-C₄-alkyl, preferably methyl or ethyl, particularly preferably ethyl, the compound according to the general formula (IV) being continuously withdrawn from the reaction.

2. A process according to claim 1,
wherein compound of the general formula (I) is used in racemic form or in the form of the (R) enantiomer; and/or
wherein in the general formula (I) the residue R¹ represents ethyl; and/or
wherein 3-hydroxybutyric acid ethyl ester (ethyl 3-hydroxybutyrate) of the formula CH₃-CH(OH)-CH₂-C(O)OC₂H₅ is used as compound of the general formula (I); and/or
wherein the reaction is carried out in the presence of an enzyme and/or a metal-containing and/or metal-based, acidic or basic catalyst, preferably in the presence of an enzyme; in particular wherein the catalyst is recycled after reaction; and/or
wherein the compound of general formula (I) is used in molar amounts, based on the hydroxyl groups of the polyglycerol (IIb), in a range from equimolar amount to a molar excess of 200 mol%, in particular in a range from equimolar amount to a molar excess of 150 mol%, preferably in a range from equimolar amount to a molar excess of 100 mol%; and/or
wherein the compound of the general formula (I) and the polyglycerol (IIb) are used in a molar ratio of compound of the general formula (I)/polyglycerol (IIb) in a range from 1:1 to 10:1, in particular in a range from 2:1 to 8:1, preferably in a range from 3:1 to 6:1; and/or
wherein the polyglycerol (IIb) is a diglycerol of formula (IIc)
HO-CH₂-CH(OH)-CH₂-O-CH₂-CH(OH)-CH₂-OH (IIc).

3. A process according to claim 1 or claim 2,
wherein the reaction product obtained is fractionated after the reaction, in particular fractionated by distillation; and/or
wherein unreacted starting compounds (I) and/or (IIb) is/are separated from the reaction product and subsequently recycled; and/or
wherein hydroxyl groups still present in the reaction product after reaction are at least partially, preferably completely, functionalized, in particular esterified; and/or wherein the reaction is followed by partial, in particular complete, functionalization, in particular esterification, of hydroxyl groups still present; in particular wherein the functionalization, in particular esterification, of the hydroxyl groups is carried out by reaction with a carboxylic acid anhydride, in particular C₂-C₃₀-carboxylic acid anhydride, preferably C₂-C₁₀-carboxylic acid anhydride, preferably C₇-carboxylic acid anhydride, in particular wherein the C₂-C₃₀-carboxylic acid anhydride, preferably C₂-C₁₀-carboxylic acid anhydride, preferably C₇-carboxylic acid anhydride, is a linear (straight-chain) or branched, saturated or mono- or polyunsaturated C₂-C₃₀-carboxylic acid anhydride, preferably C₂-C₁₀-carboxylic acid anhydride, preferably C₇-carboxylic acid anhydride.

4. A reaction product, namely a 3-hydroxybutyric acid polyglycerol ester, obtainable according to the process of any one of claims 1 to 3.

5. A 3-hydroxybutyric acid polyol ester, namely 3-hydroxy¬butyric acid polyglycerol ester,
wherein the 3-hydroxybutyric acid polyol ester has the general formula (IIIb)
R²O-CH₂-CH(OR²)-CH₂-[O-CH₂-CH(OR²)-CH₂]ₚ-OR² (IIIb)
where in the general formula (illb)
• the variable p represents an integer from 1 to 4, in particular 1 or 2, preferably 1,
• R², independently of each other, represents hydrogen, a radical CH₃-CH(OH)-CH₂-C(O)- or a radical CH₃-CH(OR³)-CH₂-C(O)- with R³ = (C₁-C₂₉-alkyl) -C(O)- or a radical (C₁-C₂₉-alkyl) - C(O) - , but with the proviso that at least one radical R², in particular at least two radicals R², is not hydrogen, and with the proviso that at least one radical R2, in particular at least two radicals R², is a radical CH₃-CH(OR³)-CH₂-C(O)- with R³ = (C₁-C₂₉-Alkyl) - C(O) -.

6. A 3-Hydroxybutyric acid polyol ester according to claim 5,
wherein in the general formula (IIIb) R², independently of one another, represents hydrogen or a radical CH₃-CH(OH)-CH₂-C(O)-, but with the proviso that at least one radical R², in particular at least two radicals R², does not represent hydrogen.

7. A 3-Hydroxybutyric acid polyol esters according to claim 5,
in which, in the general formula (IIIb), R² represents, independently of one another a radical CH₃-CH(OH)-CH₂-C(O)- or a radical CH₃-CH(OR³)-CH₂-C(O)-where R³ = (C₁-C₂₉-Alkyl) - C(O) - or a radical (C₁-C₂₉-Alkyl) - C(O) - , but with the proviso, that at least one radical R2, in particular at least two radicals R2, is a radical CH₃-CH(OH)-CH₂-C(O)- or a radical CH₃-CH(OR³)-CH₂-C(O)- where R³ = (C₁-C₂₉-Alkyl) - C(O) -.

8. A 3-hydroxybutyric acid polyol ester according to any one of claims 5 to 7,
wherein the 3-hydroxybutyric acid polyol ester of the general formula (IIIc)
R²O-CH₂-CH(OR²)-CH₂-O-CH₂-CH(OR²)-CH₂-OR² (IIIc)
in which general formula (IIIc) R² represents, independently of each other hydrogen, a radical CH₃-CH(OH)-CH₂-C(O)- or a radical CH₃-CH(OR³)-CH₂-C(O)-where R³ = (C₁-C₂₉-Alkyl) - C(O) -or a radical (C₁-C₂₉-Alkyl) - C(O) -, but with the proviso that at least one radical R², in particular at least two radicals R², is not hydrogen, and with the proviso that at least one radical R², in particular at least two radicals R², is a radical CH₃-CH(OH)-CH₂-C(O)- or a radical CH₃-CH(OR³)-CH₂-C(O)- where R³ = (C₁-C₂₉-Alkyl) - C(O) -.

9. A 3-Hydroxybutyric acid polyol ester according to claim 8,
in which general formula (IIIc) R², independently of one another, represents hydrogen or a radical CH₃-CH(OH)-CH₂-C(O)-, but with the proviso that at least one radical R², in particular at least two radicals R2, does not represent hydrogen.

10. A 3-Hydroxybutyric acid polyol esters according to claim 8,
in which, in the general formula (IIIc), R² represents, independently of one another a radical CH₃-CH(OH)-CH₂-C(O)- or a radical CH₃-CH(OR³)-CH₂-C(O)-where R³ = (C₁-C₂₉-Alkyl) - C(O) - or a radical ((C₁-C₂₉-Alkyl) - C(O) -, but with the proviso, that at least one radical R², in particular at least two radicals R², is a radical CH₃-CH(OH)-CH₂-C(O)- or a radical CH₃-CH(OR³)-CH₂-C(O)- where R³ = (C₁-C₂₉-Alkyl) - C(O) -.

11. A mixture comprising at least two or three mutually different 3-hydroxybutyric acid polyol esters, namely 3-hydroxybutyric acid polyglycerol esters, as defined in any one of claims 5 to 10.

12. A pharmaceutical composition, in particular a drug or medicament, comprising a reaction product according to claim 4 and/or a 3-hydroxybutyric acid polyol ester according to any one of claims 5 to 10 and/or a mixture according to claim 11.

13. A reaction product according to claim 4 and/or A 3-hydroxybutyric acid polyol ester according to any one of claims 5 to 10 and/or mixture according to claim 11 for use in the prophylactic and/or therapeutic treatment of diseases of the human or animal body, in particular diseases in connection with a disturbance of the energy metabolism, in particular keto-body metabolism, such as in particular craniocerebral trauma, stroke, hypoxia, cardiovascular diseases such as myocardial infarction, refeeding syndrome, anorexia, epilepsy, neurodegenerative diseases such as dementia, Alzheimer's disease, Parkinson's disease, multiple sclerosis and amyotrophic lateral sclerosis, lipid metabolic diseases such as glucose transporter defect (GLUT1 defect), VL-FAOD and mitochondrio¬pathies such as mitochondrial thiolase defect, Huntington's disease, cancers such as T-cell lymphomas, astrocytomas and glioblastomas, HIV, rheumatic diseases such as rheumatoid arthritis and arthritis urica, gastrointestinal diseases such as inflammatory bowel diseases, especially ulcerative colitis and Crohn's disease, lyosomal storage diseases such as sphingolipidoses, especially Niemann-Pick disease, diabetes mellitus, and effects or side-effects of chemotherapy.

14. A food and/or food product comprising a reaction product according to claim 4 and/or a 3-hydroxybutyric acid polyol ester according to any one of claims 5 to 10 and/or a mixture according to claim 11.

15. Non-therapeutic use of a reaction product according to claim 4 and/or of a 3-hydroxybutyric acid polyol ester according to any one of claims 5 to 10 and/or of a mixture according to claim 11 in a food and/or food product.

## Revendications

1. Procédé de préparation d'esters de polyols, à savoir d'esters de polyglycérol, de l'acide 3-hydroxybutyrique (acide béta-hydroxybutyrique, BHB ou 3-BHB),
où au moins un composé de formule générale (I)
CH₃-CH(OH)-CH₂-C(O)OR¹ (I)
où, dans la formule générale (I), le radical R¹ représente l'hydrogène ou un alkyle en C₁-C₄, en particulier un alkyle en C₁-C₄, de préférence le méthyle ou l'éthyle, de manière particulièrement préférée l'éthyle,
est mis en réaction avec au moins un polyglycérol de formule générale (Ilb)
HO-CH₂-CH(OH)-CH₂-[O-CH₂-CH(OH)-CH₂]ₚ-OH (IIb)
où, dans la formule générale (Ilb), la variable p représente un nombre entier de 1 à 4, en particulier 1 ou 2, de préférence 1,
la réaction étant effectuée en l'absence de solvants et/ou sans aucun solvant, et
la réaction étant effectuée en présence d'un catalyseur,
de sorte que l'on obtient, comme produit de réaction, un ou plusieurs polyesters d'acide 3-hydroxybutyrique (III), à savoir des esters de polyglycérol d'acide 3-hydroxybutyrique,
la réaction donnant simultanément le composé selon la formule générale (IV)
R¹-OH (IV)
où, dans la formule générale (IV), le radical R¹ représente l'hydrogène ou un alkyle en C₁-C₄, en particulier un alkyle en C₁-C₄, de préférence le méthyle ou l'éthyle, de manière particulièrement préférée l'éthyle, le composé selon la formule générale (IV) étant retiré en continu de la réaction.

2. Procédé selon la revendication 1,
où le composé de formule générale (I) est utilisé sous forme racémique ou sous forme d'énantiomère (R); et/ou
où, dans la formule générale (I), le reste R¹ représente un éthyle; et/ou
où on utilise, comme composé de formule générale (I), l'ester éthylique de l'acide 3-hydroxybutyrique (3-hydroxybutyrate d'éthyle) de formule CH₃-CH(OH)-CH₂-C(O)OC₂H₅ ; et/ou
où la réaction est effectuée en présence d'une enzyme et/ou d'un catalyseur acide ou basique contenant un métal et/ou à base de métal, de préférence en présence d'une enzyme; en particulier, le catalyseur étant recyclé après la réaction; et/ou
où le composé de formule générale (I) est utilisé, par rapport aux groupes hydroxyle du polyglycérol (Ilb), en quantités molaires dans une plage allant d'une quantité équimolaire jusqu'à un excès molaire de 200 % en moles, en particulier dans une plage allant d'une quantité équimolaire jusqu'à un excès molaire de 150 % en moles, de préférence dans une plage allant d'une quantité équimolaire jusqu'à un excès molaire de 100 % en moles; et/ou
où le composé de formule générale (I) et le polyglycérol (Ilb) sont utilisés dans un rapport molaire composé de formule générale (I)/polyglycérol (Ilb) dans une plage de 1:1 à 10:1, en particulier dans une plage de 2:1 à 8:1, de préférence dans une plage de 3:1 à 6:1; et/ou
où le polyglycérol (Ilb) est un diglycérol de formule (Ilc)
HO-CH₂-CH(OH)-CH₂-O-CH₂-CH(OH)-CH₂-OH (IIc) .

3. Procédé selon la revendication 1 ou la revendication 2,
où le produit de réaction obtenu est fractionné après la réaction, en particulier fractionné par distillation; et/ou
où les composés de départ (I) et/ou (IIb) n'ayant pas réagi sont séparés du produit de réaction et ensuite recyclés; et/ou
où les groupes hydroxyle encore présents dans le produit de réaction après la réaction sont au moins partiellement, de préférence complètement, fonctionnalisés, en particulier estérifiés; et/ou où la réaction est suivie d'une fonctionnalisation, en particulier d'une estérification, partielle, en particulier complète, des groupes hydroxyle encore présents; en particulier où la fonctionnalisation, en particulier l'estérification, des groupes hydroxyle est effectuée par réaction avec un anhydride d'acide carboxylique, en particulier un anhydride d'acide carboxylique en C₂-C₃₀, de préférence un anhydride d'acide carboxylique en C₂-C₁₀, de préférence un anhydride d'acide carboxylique en C₇, en particulier où l'anhydride d'acide carboxylique en C₂-C₃₀, de préférence l'anhydride d'acide carboxylique en C₂-C₁₀, de préférence l'anhydride d'acide carboxylique en C₇, est un anhydride d'acide carboxylique en C₂-C₃₀ linéaire (à chaîne droite) ou ramifié, saturé ou mono- ou polyinsaturé, de préférence l'anhydride d'acide carboxylique en C₂-C₁₀, de préférence l'anhydride d'acide carboxylique en C₇.

4. Produit de réaction, à savoir l'ester de polyglycérol de l'acide 3-hydroxybutyrique, pouvant être obtenu selon le procédé selon l'une quelconque des revendications 1 à 3.

5. Le polyolester de l'acide 3-hydroxybutyrique, à savoir l'ester de polyglycérol de l'acide 3-hydroxybutyrique,
où ledit ester de polyol de l'acide 3-hydroxybutyrique est représenté par la formule générale (IIIb)
R²O-CH₂-CH(OR²)-CH₂-[O-CH₂-CH(OR²)-CH₂]ₚ-OR² (IIIb)
où, dans la formule générale (IIIb)
• la variable p représente un nombre entier de 1 à 4, notamment 1 ou 2, de préférence 1,
• R², indépendamment les uns des autres, représente: l'hydrogène, un radical CH₃-CH(OH)-CH₂-C(O)- ou un radical CH₃-CH(OR³)-CH₂-C(O)- avec R³ = (C₁-C₂₉-alkyl)- C(O) - ou un radical (C₁-C₂₉-alkyl)-C(O)- ,
à condition toutefois qu'au moins un radical R², notamment au moins deux radicaux R², ne représente pas un hydrogène, et à condition qu'au moins un radical R², en particulier au moins deux radicaux R², représente un radical CH₃-CH(OH)-CH₂-C(O)-ou un radical CH₃-CH(OR³)-CH₂-C(O)- avec R³ = (C₁-C₂₉-alkyl)-C(O)- .

6. Polyester d'acide 3-hydroxybutyrique selon la revendication 5,
où, dans la formule générale (IIIb), les R², indépendamment les uns des autres, représentent l'hydrogène ou un radical CH₃-CH(OH)-CH₂-C(O)-, à condition toutefois qu'au moins un radical R², en particulier au moins deux radicaux R², ne représente pas l'hydrogène.

7. Polyester d'acide 3-hydroxybutyrique selon la revendication 5,
où, dans la formule générale (IIIb), R² représente, indépendamment: un radical CH₃-CH(OH)-CH₂-C(O)- ou un radical CH₃-CH(OR³)-CH₂-C(O)- avec R³ = (C₁-C₂₉-alkyl)-C(O)- ou un radical (C₁-C₂₉-alkyl)-C(O)- , à condition toutefois qu'au moins un radical R², en particulier au moins deux radicaux R², représente un radical CH₃-CH(OH)-CH₂-C(O)- ou un radical CH₃-CH(OR³)-CH₂-C(O)- avec R³ = (C₁-C₂₉-alkyl)-C(O)- .

8. Polyester de l'acide 3-hydroxybutyrique selon l'une quelconque des revendications 5 à 7,
où ledit ester de polyol de l'acide 3-hydroxybutyrique est représenté par la formule générale (IIIc),
R²O-CH₂-CH(OR²)-CH₂-O-CH₂-CH(OR²)-CH₂-OR² (IIIc)
où, dans la formule générale (IIIc), R² représente indépendamment: l'hydrogène, un radical CH₃-CH(OH)-CH₂-C(O)- ou un radical CH₃-CH(OR³)-CH₂-C(O)- avec R³ = (C₁-C₂₉-alkyl)-C(O)- ou un radical (C₁-C₂₉-alkyl)-C(O)-, à condition toutefois qu'au moins un radical R², notamment au moins deux radicaux R², ne représente pas un hydrogène, et à condition qu'au moins un radical R², en particulier au moins deux radicaux R², représente un radical CH₃-CH(OH)-CH₂-C(O)- ou un radical CH₃-CH(OR³)-CH₂-C(O)- avec R³ = (C₁-C₂₉-alkyl)-C(O)-.

9. Polyester d'acide 3-hydroxybutyrique selon la revendication 8,
où, dans la formule générale (IIIc), R² représente, indépendamment l'un de l'autre, de l'hydrogène ou un radical CH₃-CH(OH)-CH₂-C(O)-, à condition toutefois qu'au moins un radical R², en particulier au moins deux radicaux R², ne représente pas de l'hydrogène.

10. Polyester d'acide 3-hydroxybutyrique selon la revendication 8,
où, dans la formule générale (IIIc), R² représente, indépendamment: un radical CH₃-CH(OH)-CH₂-C(O)- ou un radical CH₃-CH(OR³)-CH₂-C(O)- avec R³ = (C₁-C₂₉-alkyl)-C(O)- ou un radical (C₁-C₂₉-alkyl)-C(O)-, à condition toutefois que , qu'au moins un radical R², en particulier au moins deux radicaux R², représente un radical CH₃-CH(OH)-CH₂-C(O)- ou un radical CH₃-CH(OR³)-CH₂-C(O)- avec R³ = (C₁-C₂₉-alkyl)- C(O) -.

11. Mélange comprenant au moins deux ou trois polyolesters d'acide 3-hydroxybutyrique différents les uns des autres, à savoir des esters d'acide 3-hydroxybutyrique et de polyglycérol, tel que défini dans l'une quelconque des revendications 5 à 10.

12. Composition pharmaceutique, en particulier un médicament, comprenant un produit de réaction selon la revendication 4 et/ou un ester de polyol et d'acide 3-hydroxybutyrique selon l'une quelconque des revendications 5 à 10 et/ou un mélange selon la revendication 11.

13. Produit de réaction selon la revendication 4 et/ou 3 polyolester de l'acide hydroxybutyrique selon l'une quelconque des revendications 5 à 10 et/ou mélange selon la revendication 11, destiné à être utilisé dans le traitement prophylactique et/ou thérapeutique de maladies du corps humain ou animal, en particulier les maladies liées à une perturbation du métabolisme énergétique, en particulier du métabolisme des corps cétoniques, telles que notamment les traumatismes crâniens, les accidents vasculaires cérébraux, les hypoxies, les maladies cardio-vasculaires telles que l'infarctus du myocarde, le syndrome d'abandon, les anorexies, l'épilepsie, les maladies neurodégénératives telles que la démence, la maladie d'Alzheimer, la maladie de Parkinson, sclérose en plaques et sclérose latérale amyotrophique, maladies du métabolisme lipidique telles que le déficit en transporteur de glucose (déficit GLUT1), VL-FAOD et les mitochondriopathies telles que le déficit en thiolase mitochondriale, la chorée de Huntington, les cancers tels que les lymphomes à cellules T, les astrocytomes et les glioblastomes, le VIH, les maladies rhumatismales telles que la polyarthrite rhumatoïde et l'arthrite urique, les maladies du tractus gastro-intestinal telles que les maladies inflammatoires chroniques de l'intestin, notamment la colite ulcéreuse et la maladie de Crohn, les maladies du stockage lyosomique telles que les sphingolipidoses, notamment la maladie de Niemann-Pick, le diabète sucré et les effets ou effets secondaires des chimiothérapies.

14. Produit alimentaire et/ou nutritionnel comprenant un produit de réaction selon la revendication 4 et/ou un ester de polyol et d'acide 3-hydroxybutyrique selon l'une quelconque des revendications 5 à 10 et/ou un mélange selon la revendication 11.

15. Utilisation non-thérapeutique d'un produit de réaction selon la revendication 4 et/ou d'un polyester d'acide 3-hydroxybutyrique selon l'une quelconque des revendications 5 à 10 et/ou d'un mélange selon la revendication 11 dans un produit alimentaire et/ou nutritionnel.
